# EUROPEAN PATENT APPLICATION

(11) **EP 4 527 929 A1**
(43) Date of publication of application: **26.03.2025**
(21) Application number: 23807703.6
(22) Date of filing: 18.05.2023
(51) Int. Cl.: C12N 15/113, A61K 31/7088, A61P 43/00, C12N 15/09, C12N 15/10

(54) **NEW STAPLE NUCLEIC ACID**

(30) Priority: 18.05.2022 JP 2022081914
(71) Applicant: National University Corporation Kumamoto University, Kumamoto-shi, Kumamoto 860-8555 (JP)
(72) Inventor: KATSUDA Yousuke, Kumamoto-shi, Kumamoto 860-8555 (JP); IHARA Toshihiro, Kumamoto-shi, Kumamoto 860-8555 (JP); KITAMURA Yusuke, Kumamoto-shi, Kumamoto 860-8555 (JP); KAMURA Takuto, Kumamoto-shi, Kumamoto 860-8555 (JP); SHIMOTAKE Katsunori, Kumamoto-shi, Kumamoto 860-8555 (JP); ITSUKI Yua, Kumamoto-shi, Kumamoto 860-8555 (JP)
(74) Representative: Plasseraud IP
(86) International application number: PCT/JP2023/018646
(87) International publication number: WO 2023/224102

(57) **Abstract**

The present invention aims to develop Staple nucleic acids capable of forming guanine quadruplex structure on a target nucleic acid even when four guanine repeat sequences are not present on the target nucleic acid. The present invention also aims to develop various use applications of existing Staple nucleic acids and Staple nucleic acids of the present invention. The present inventors have solved the above-described problems by developing an oligonucleotide (second generation-type Staple nucleic acid) which can supply guanine repeat sequences via an oligonucleotide, so that a guanine quadruplex structure can be formed on a target nucleic acid by a total of four guanine repeat sequences, consisting of guanine repeat sequences on the target nucleic acid and guanine repeat sequences on the oligonucleotide. The present invention also has developed novel use applications of the first generation-type Staple nucleic acids and that of the second generation-type Staple nucleic acids.

## Description

### Technical Field

The present invention relates to development of a novel type of Staple nucleic acid. The present invention also relates to development of a use application of the conventional Staple nucleic acid and a novel Staple nucleic acid.

### Background Art

As gene expression suppression techniques, various tools such as microRNA and siRNA are known in the art (Non Patent Literature 1 and 2). If the sequence information of a target gene is known, siRNA can be relatively easily designed, and it is possible to strongly suppress gene expression by selecting a plurality of target sequences for one target mRNA, with reference to existing reports on the target gene.

In any of such techniques, since the gene expression-suppressing function is exerted by a simple mechanism of binding to mRNA, unexpected effects (off-target effects) are unavoidable (Non Patent Literature 3), and there may be problems to be solved for using such gene expression-suppressing function inside the human body.

Due to the current advancement in bioinformatics technology, it is now possible to minimize the suppression of expression of unintended genes (off-target effects) can be minimized. However, from a standpoint of pharmaceutical development, it cannot be said that siRNA development have made good progress. The reason is that nucleic acid medicines require the use of modified nucleic acids. It is known to cause a problem that the use of modified nucleic acids may not exhibit a desired protein translation-suppressing effect by markedly decreasing the microRNA effect and siRNA activity (Non Patent Literature 4).

In order to solve these problems, various artificially modified nucleic acids have been synthesized, but in the present circumstances, development of artificial modified nucleic acids with high versatility as pharmaceutical products is still underway. The leading cause of difficulty in development of artificially modified nucleic acids is the fact that siRNA requires interlocking with an enzymatic reaction having target mRNA-cleaving activity, that is, while nuclease resistance is acquired by chemically modifying nucleic acids, the ability to be recognized as a substrate for enzymatic reaction is lost.

For solving these problems, the present inventors so far developed a Staple nucleic acid having the function of binding to a target mRNA sequence in a sequence specific manner, and using four guanine repeat sequences present on target mRNA to induce formation of a guanine quadruplex structure, and a method for suppressing protein translation reaction by inhibiting peptide synthesis reaction of ribosomes using the guanine quadruplex structure induced by the Staple nucleic acid (Patent Literature 1).

The conventional Staple nucleic acids require four guanine repeat sequences on target mRNA, and can form a guanine quadruplex structure only for the target mRNA having a structure meeting this requirement.

### Citation List

### Patent Literature

Patent Literature 1: WO 2020/085510

### Non Patent Literature

Non Patent Literature 1: Fire, A., et al., Nature 1998, 391, 806
Non Patent Literature 2: Ambros, V. Nature 2004, 431, 350
Non Patent Literature 3: Jackson, A.L. and Linsley, P.S. Nature review. Drug discovery 2010, 9, 57
Non Patent Literature 4: Jackson, A.L., et al., Rna 2006, 12, 1197

### Summary of Invention

### Technical Problem

The present invention aims to develop Staple nucleic acids capable of forming guanine quadruplex structure on a target nucleic acid even when four guanine repeat sequences are not present on the target nucleic acid. The present invention also aims to develop various use applications of existing Staple nucleic acids and Staple nucleic acids of the present invention.

### Solution to Problem

The present inventors have solved the above-described problems by developing a Staple nucleic acid (second generation-type Staple nucleic acid) which can supply guanine repeat sequences via an oligonucleotide, so that a guanine quadruplex structure can be formed on a target nucleic acid by a total of four guanine repeat sequences, consisting of guanine repeat sequences on the target nucleic acid and guanine repeat sequences on the oligonucleotide. The present invention also has developed novel use applications of the first generation-type Staple nucleic acids and that of the second generation-type Staple nucleic acids.

More specifically, the present application provides the following embodiments for solving the foregoing problems.
[1]: A G supply-type oligonucleotide comprising: a first nucleotide sequence and a second nucleotide sequence, which target a nucleotide sequence portion containing one to three guanine repeat sequences on a target nucleic acid, and each hybridize with a nucleotide sequence on the 5' side or 3' side of the guanine repeat sequences; and three to one guanine repeat sequences depending on the number of guanine repeat sequences on the target nucleic acid,
   wherein the G supply-type oligonucleotide changes the conformation of the target nucleic acid through hybridization of the first nucleotide sequence and the second nucleotide sequence with the target nucleic acid, decreases a spatial distance between a total of four guanine repeat sequences consisting of the guanine repeat sequences on the target nucleic acid and the guanine repeat sequences on the G supply-type oligonucleotide, to form a guanine quadruplex structure composed of the four guanine repeat sequences.
[2]: The G supply-type oligonucleotide according to [1], wherein the first nucleotide sequence and the second nucleotide sequence of the G supply-type oligonucleotide hybridize with
   - a nucleotide sequence portion on the 5' side or 3' side of a guanine repeat sequence on the 3' terminal side of the guanine repeat sequences on the target nucleic acid, and
   - a nucleotide sequence portion on the 5' side or 3' side of a guanine repeat sequence on the 5' terminal side of the guanine repeat sequences on the target nucleic acid.
[3]: The G supply-type oligonucleotide according to [1] or [2], which is composed of DNA, RNA, a modified nucleic acid, or a combination thereof.
[4]: A pharmaceutical composition comprising a G supply-type oligonucleotide comprising: a first nucleotide sequence and a second nucleotide sequence which target a nucleotide sequence portion containing one to three guanine repeat sequences on a target nucleic acid, and each hybridize with a nucleotide sequence on the 5' side or 3' side of the guanine repeat sequences; and three to one guanine repeat sequences depending on the number of guanine repeat sequences on the target nucleic acid,
   wherein the G supply-type oligonucleotide changes the conformation of the target nucleic acid through hybridization of the first nucleotide sequence and the second nucleotide sequence with the target nucleic acid, decreases a spatial distance between a total of four guanine repeat sequences consisting of the guanine repeat sequences on the target nucleic acid and the guanine repeat sequences on the G supply-type oligonucleotide, to form a guanine quadruplex structure composed of the four guanine repeat sequences.
[5]: The pharmaceutical composition according to [4], wherein the first nucleotide sequence and the second nucleotide sequence of the G supply-type oligonucleotide hybridize with
   - a nucleotide sequence portion on the 5' side or 3' side of a guanine repeat sequence on the 3' terminal side of the guanine repeat sequences on the target nucleic acid, and
   - a nucleotide sequence portion on the 5' side or 3' side of a guanine repeat sequence on the 5' terminal side of the guanine repeat sequences on the target nucleic acid.
[6]: The pharmaceutical composition according to [4] or [5], wherein the G supply-type oligonucleotide is composed of DNA, RNA, a modified nucleic acid, or a combination thereof.
[7]: A method for producing a G supply-type oligonucleotide comprising: a first nucleotide sequence and a second nucleotide sequence which target a nucleotide sequence portion containing one to three guanine repeat sequences on a target nucleic acid, and each hybridize with a nucleotide sequence on the 5' side or 3' side of the guanine repeat sequences; and three to one guanine repeat sequences depending on the number of guanine repeat sequences on the target nucleic acid,
   the method comprising designing and synthesizing the G supply-type oligonucleotide such that the first nucleotide sequence and the second nucleotide sequence of the G supply-type oligonucleotide hybridize with the target nucleic acid, and the G supply-type oligonucleotide is folded so as to decrease a spatial distance between a total of four guanine repeat sequences consisting of the guanine repeat sequences on the target nucleic acid and the guanine repeat sequences on the G supply-type oligonucleotide.
[8]: The method for producing a G supply-type oligonucleotide according to [7], wherein the first nucleotide sequence and the second nucleotide sequence of the G supply-type oligonucleotide hybridize with
   - a nucleotide sequence portion on the 5' side or 3' side of a guanine repeat sequence on the 3' terminal side of the guanine repeat sequences on the target nucleic acid, and
   - a nucleotide sequence portion on the 5' side or 3' side of a guanine repeat sequence on the 5' terminal side of the guanine repeat sequences on the target nucleic acid.
[9]: The method for producing a G supply-type oligonucleotide according to [7] or [8], wherein the G supply-type oligonucleotide is composed of DNA, RNA, a modified nucleic acid, or a combination thereof.
[10]: A method for downregulating protein expression from a target nucleic acid, using a G supply-type oligonucleotide comprising: a first nucleotide sequence and a second nucleotide sequence which target a nucleotide sequence portion containing one to three guanine repeat sequences on a target nucleic acid, and each hybridize with a nucleotide sequence on the 5' side or 3' side of the guanine repeat sequences; and three to one guanine repeat sequences depending on the number of guanine repeat sequences on the target nucleic acid,
   to change the conformation of the target nucleic acid through hybridization of the first nucleotide sequence and the second nucleotide sequence with the target nucleic acid, to decrease a spatial distance between a total of four guanine repeat sequences consisting of the guanine repeat sequences on the target nucleic acid and the guanine repeat sequences on the G supply-type oligonucleotide, to form a guanine quadruplex structure composed of the four guanine repeat sequences, by the G supply-type oligonucleotide.
[11]: The method for downregulating protein expression according to [10], wherein the first nucleotide sequence and the second nucleotide sequence of the G supply-type oligonucleotide hybridize with
   - a nucleotide sequence portion on the 5' side or 3' side of a guanine repeat sequence on the 3' terminal side of the guanine repeat sequences on the target nucleic acid, and
   - a nucleotide sequence portion on the 5' side or 3' side of a guanine repeat sequence on the 5' terminal side of the guanine repeat sequences on the target nucleic acid.
[12]: The method for downregulating protein expression according to [10] or [11], wherein the G supply-type oligonucleotide is composed of DNA, RNA, a modified nucleic acid, or a combination thereof.
[13]: The method for downregulating protein expression according to [10] or [11], wherein the downregulation of protein expression occurs through suppression of reverse transcription reaction or suppression of protein translation reaction.
[14]: A kit for downregulating protein expression, comprising a G supply-type oligonucleotide comprising: a first nucleotide sequence and a second nucleotide sequence which target a nucleotide sequence portion containing one to three guanine repeat sequences on a target nucleic acid, and each hybridize with a nucleotide sequence on the 5' side or 3' side of the guanine repeat sequences; and three to one guanine repeat sequences depending on the number of guanine repeat sequences on the target nucleic acid,
   wherein the G supply-type oligonucleotide downregulates protein expression from the target nucleic acid by changing the conformation of the target nucleic acid through hybridization of the first nucleotide sequence and the second nucleotide sequence with the target nucleic acid, decreasing a spatial distance between a total of four guanine repeat sequences consisting of the guanine repeat sequences on the target nucleic acid and the guanine repeat sequences on the G supply-type oligonucleotide, to form a guanine quadruplex structure composed of the four guanine repeat sequences.
[15]: The kit for downregulating protein expression according to [14], wherein the first nucleotide sequence and the second nucleotide sequence of the G supply-type oligonucleotide hybridize with
   - a nucleotide sequence portion on the 5' side or 3' side of a guanine repeat sequence on the 3' terminal side of the guanine repeat sequences on the target nucleic acid, and
   - a nucleotide sequence portion on the 5' side or 3' side of a guanine repeat sequence on the 5' terminal side of the guanine repeat sequences on the target nucleic acid.
[16]: The kit for downregulating protein expression according to [14] or [15], wherein the downregulation of protein expression occurs through suppression of reverse transcription reaction or suppression of protein translation reaction.
[17]: A method for producing a modified protein from a target nucleic acid using an oligonucleotide comprising: a first nucleotide sequence and a second nucleotide sequence, which target a nucleotide sequence portion containing one to four guanine repeat sequences on a target nucleic acid, and each hybridize with a nucleotide sequence on the 5' side or 3' side of the guanine repeat sequences; and three to zero guanine repeat sequences depending on the number of guanine repeat sequences on the target nucleic acid,
   wherein the oligonucleotide forms a loop portion on a part of the target nucleic acid, by changing the conformation of the target nucleic acid through hybridization of the first nucleotide sequence and the second nucleotide sequence with the target nucleic acid, decreasing a spatial distance between a total of four guanine repeat sequences consisting of guanine repeat sequences on the target nucleic acid and guanine repeat sequences on the oligonucleotide, to form a guanine quadruplex structure composed of the four guanine repeat sequences, and ribosomes shunt over the loop portion.
[18]: The method for producing a modified protein according to [17], wherein the first nucleotide sequence and the second nucleotide sequence of the oligonucleotide hybridize with
   - a nucleotide sequence portion on the 5' side or 3' side of a guanine repeat sequence on the 3' terminal side of the guanine repeat sequences on the target nucleic acid, and
   - a nucleotide sequence portion on the 5' side or 3' side of a guanine repeat sequence on the 5' terminal side of the guanine repeat sequences on the target nucleic acid.
[19]: The method for producing a modified protein according to [17] or [18], wherein the oligonucleotide is composed of DNA, RNA, a modified nucleic acid, or a combination thereof.
[20]: A method for producing a fused protein of a translated product fused with a sub-sequence of a first target nucleic acid and a sub-sequence of a second target nucleic acid, using an oligonucleotide comprising:
   a first nucleotide sequence which targets a nucleotide sequence portion containing one to two guanine repeat sequences on the first target nucleic acid, and hybridizes with a nucleotide sequence on the 5' side or 3' side of the guanine repeat sequences,
   a second nucleotide sequence which targets a nucleotide sequence portion containing one to two guanine repeat sequences on the second target nucleic acid, and hybridizes with a nucleotide sequence on the 5' side or 3' side of the guanine repeat sequences, and
   two to zero guanine repeat sequences depending on the number of the guanine repeat sequences on the first target nucleic acid and the number of the guanine repeat sequences on the second target nucleic acid,
   wherein the oligonucleotide hybridizes with the first target nucleic acid through its first nucleotide sequence and with the second target nucleic acid through its second nucleotide sequence, changes the conformation of the target nucleic acids, decreases a spatial distance between a total of four guanine repeat sequences consisting of one to two guanine repeat sequences on the first target nucleic acid, one to two guanine repeat sequences on the second target nucleic acid and two to zero guanine repeat sequences on the oligonucleotide, to form a guanine quadruplex structure composed of the four guanine repeat sequences, and ribosomes shunt between the first target nucleic acid and the second target nucleic acid.
[21]: The method for producing a fused protein according to [20], wherein the first nucleotide sequence and the second nucleotide sequence of the oligonucleotide hybridize with
   - a nucleotide sequence portion on the 5' side or 3' side of a guanine repeat sequence on the 3' terminal side of the guanine repeat sequences on the target nucleic acid, and
   - a nucleotide sequence portion on the 5' side or 3' side of a guanine repeat sequence on the 5' terminal side of the guanine repeat sequences on the target nucleic acid.
[22]: The method for producing a fused protein according to [20] or [21], wherein the oligonucleotide is composed of DNA, RNA, a modified nucleic acid, or a combination thereof.
[23]: A method for stabilizing a target nucleic acid, using an oligonucleotide comprising: a first nucleotide sequence and a second nucleotide sequence which target a nucleotide sequence portion containing one to four guanine repeat sequences on a target nucleic acid, and each hybridize with a nucleotide sequence on the 5' side or 3' side of the guanine repeat sequences; and three to zero guanine repeat sequences depending on the number of guanine repeat sequences on the target nucleic acid,
   wherein the oligonucleotide inhibits binding of a nucleolytic enzyme to the target nucleic acid or inhibits functions of the nucleolytic enzyme by changing the conformation of the target nucleic acid through hybridization of the first nucleotide sequence and the second nucleotide sequence with the target nucleic acid, decreasing a spatial distance between a total of four guanine repeat sequences consisting of guanine repeat sequences on the target nucleic acid and guanine repeat sequences on the oligonucleotide, to form a guanine quadruplex structure composed of the four guanine repeat sequences.
[24]: The method for stabilizing a target nucleic acid according to [23], wherein the first nucleotide sequence and the second nucleotide sequence of the oligonucleotide hybridize with
   - a nucleotide sequence portion on the 5' side or 3' side of a guanine repeat sequence on the 3' terminal side of the guanine repeat sequences on the target nucleic acid, and
   - a nucleotide sequence portion on the 5' side or 3' side of a guanine repeat sequence on the 5' terminal side of the guanine repeat sequences on the target nucleic acid.
[25]: The method for stabilizing a target nucleic acid according to [23] or [24], wherein the oligonucleotide is composed of DNA, RNA, a modified nucleic acid, or a combination thereof.

### Advantageous Effects of Invention

The present invention can develop and provide a novel oligonucleotide (second generation-type Staple nucleic acid) which can supply guanine repeat sequences via an oligonucleotide, so that a guanine quadruplex structure can be formed on a target nucleic acid by a total of four guanine repeat sequences consisting of the guanine repeat sequences on the target nucleic acid and the guanine repeat sequences on the oligonucleotide. The present invention can also provide novel use applications of the first generation-type Staple nucleic acids and that of the second generation-type Staple nucleic acids.

### Brief Description of Drawings

[Figure 1] Figure 1 shows formation of a guanine quadruplex structure when the binding mode of a G supply-type Staple nucleic acid is changed.
[Figure 2] Figure 2 shows construction of a guanine quadruplex structure using a G supply-type Staple nucleic acid in 2+1 100nt, 1+1 100nt_mut 1 or 1+1 100nt_mut 2, which are model sequences. Here, (A) shows the results of fluorescence measurement using Thioflavin T on 2+1 100nt RNA, (B) shows the result of fluorescence measurement using Thioflavin T on 1+1 100nt_mut 1 RNA, and (C) shows the result of fluorescence measurement using Thioflavin T on 1+1 100nt_mut 2 RNA.
[Figure 3] Figure 3 shows the identification of a location at which a guanine quadruplex structure is formed in each target nucleic acid sequence. Here, Figure 3 (A) shows the results of stop assay in the 1+1 100nt RNA sequence, and Figure 3 (B) shows the results of stop assay in the TRPC6 5'UTR RNA sequence.
[Figure 4] Figure 4 shows the results of examining the length and T linkers of a G supply-type Staple nucleic acid in the TRPC6 5'UTR sequence. Here, Figure 4 (A) shows the results of stop assay when the number of T linkers in the G supply-type Staple nucleic acid is changed, and Figure 4 (B) shows the results of stop assay when the length of the G supply-type Staple nucleic acid is changed.
[Figure 5] Figure 5 shows the results of evaluating the effects of a guanine quadruplex structure constructed by a G supply-type Staple nucleic acid on protein translation reaction. Here, Figure 5 (A) shows an outline of each nucleic acid template used for in *in vitro* translation, and Figure 5 (B) shows the results (n=2) of *in vitro* translation in the TRPC6 5'UTR sequence.
[Figure 6] Figure 6 shows the results of evaluating whether the stability of a target nucleic acid is improved by the presence of a Staple nucleic acid.
[Figure 7] Figure 7 shows the results of comparing the GFP signal intensities of PTC GFP (no Staple nucleic acid), Δ (delta) type1 GFP (no Staple nucleic acid), PTC GFP (Staple nucleic acid 1) and PTC GFP (Staple nucleic acid 2) where the GFP signal strength when GFP mRNA is translated is defined as 100.
[Figure 8-1] Figure 8-1 shows that the presence of a Staple nucleic acid markedly improved the stability of a target nucleic acid.
[Figure 8-2] Figure 8-2 shows that, when cells were used, the stability of a target nucleic acid is improved by the presence of a Staple nucleic acid, resulting in upregulation of protein expression in the cells.
[Figure 9-1] Figure 9-1 shows that the stability of a target nucleic acid is markedly improved by the presence of a Staple nucleic acid.
[Figure 9-2] Figure 9-2 shows that, when cells were used, the stability of a target nucleic acid is improved by the presence of a Staple nucleic acid, resulting in upregulation of protein expression in the cells.
[Figure 10] Figure 10 shows that shunting of ribosomes in a single molecule can be generated by a Staple nucleic acid of the present invention.
[Figure 11] Figure 11 shows that shunting of ribosomes between two molecules can be generated a Staple nucleic acid of the present invention.

### Description of Embodiments

The terms used in the present invention are defined as follows:
(a) Staple nucleic acid: an oligonucleotide having the function of hybridizing with two sequence region on a target nucleic acid in a sequence specific manner, changing the conformation of the target nucleic acid, and inducing formation of a guanine quadruplex structure using guanine repeat sequences present on the target nucleic acid. There are a non-G supply-type Staple nucleic acid (non-G supply-type oligonucleotide, also referred to as first generation-type Staple nucleic acid) described later, and a G supply-type Staple nucleic acid (G supply-type oligonucleotide, also referred to as second generation-type Staple nucleic acid).
(b) Non-G supply-type Staple nucleic acid: a type of Staple nucleic acid which does not contain guanine repeat sequences in the Staple nucleic acid itself, and is prepared with reference to the Staple nucleic acid disclosed in Patent Document 1. The Non-G supply-type Staple nucleic acid refers to an oligonucleotide having the function of hybridizing with two sequence regions on a target nucleic acid in a sequence specific manner, changing the conformation of the target nucleic acid, decreasing a spatial distance between four guanine repeat sequences present on the target nucleic acid, and inducing formation of a guanine quadruplex structure. The non-G supply-type Staple nucleic acid in the present invention also referred to as a non-G supply-type oligonucleotide, or a first generation-type Staple nucleic acid.
(c) G supply-type Staple nucleic acid: a type of Staple nucleic acid which contains at least one guanine repeat sequence in the Staple nucleic acid itself, and is newly disclosed in the present invention. The G supply-type Staple nucleic acid refers to an oligonucleotide having the function of hybridizing with two sequence regions on a target nucleic acid in a sequence specific manner, changing the conformation of the target nucleic acid, decreasing a spatial distance between a total of four guanine repeat sequences consisting of the guanine repeat sequences present on the target nucleic acid and the guanine repeat sequences present on the G supply-type Staple nucleic acid, and inducing formation of a guanine quadruplex structure. The G supply-type Staple nucleic acid in the present invention also referred to as a G supply-type oligonucleotide, or a second generation-type Staple nucleic acid.
(d) Nucleic acid: the nucleic acid constituting the Staple nucleic acid in the present invention refers to a naturally occurring nucleic acid, or a nucleic acid that does not exist in nature (non-natural nucleic acid). The naturally occurring nucleic acid refers to DNA or RNA consisting of nucleobases (i.e., adenine (a), guanine (g), cytosine (c), thymine (t) and uracil (u)). The nucleic acid that does not exist in nature (non-natural nucleic acid) refers to a nucleic acid whose physical properties have been changed by applying a modification to nucleic-acid base/sugar/phosphoric acid diester portions thereof, and is also referred to as a modified nucleic acid.

### Structure of first generation-type Staple nucleic acid

The first generation-type Staple nucleic acid in the present invention refers to a Staple nucleic acid prepared with reference to the Staple nucleic acid disclosed in Patent Literature 1. That is, the first generation-type Staple nucleic acid is structurally characterized in that the Staple nucleic acid itself does not contain guanine repeat sequence(s) which form a guanine quadruplex structure.

The first generation-type Staple nucleic acid in the present invention refers to an oligonucleotide comprising a first nucleotide sequence and a second nucleotide sequence, which target a nucleotide sequence portion containing four guanine repeat sequences on a target nucleic acid, and each hybridize with a nucleotide sequence adjacent to (i.e., on the 5' side or 3' side of) any two of the guanine repeat sequences, wherein the oligonucleotide has the function of changing the conformation of the target nucleic acid through hybridization of the first nucleotide sequence and the second nucleotide sequence with the target nucleic acid, decreasing a spatial distance between the four guanine repeat sequences on the target nucleic acid, and forming a guanine quadruplex structure composed of the four guanine repeat sequences.

In general, the guanine quadruplex structure in cells is a stable nucleic acid higher-order structure formed when four guanine repeat sequences each having 3 or more bases are present in close spatial arrangement of less than 7 bases. The present inventors of the present invention have found that even when guanine repeat sequences are present at distant locations with spacing of 7 or more bases on the target nucleic acid, by using an oligonucleotide containing a first nucleotide sequence and a second nucleotide sequence appropriately designed according to the present invention, the guanine repeat sequences present on the target nucleic acid can be artificially brought into spatial proximity to induce an artificial guanine quadruplex structure in the target nucleic acid.

As used herein, the guanine repeat sequence in a target nucleic acid to which the first generation-type Staple nucleic acid binds refers to a consecutive sequence containing 3 or more guanine bases present on the target nucleic acid, or a sequence containing 3 or more guanine bases including a nucleotide sequence forming a bulge structure between guanines. Examples of the specific guanine repeat sequence include GGG, and GNGG (N may be any bases, and the number thereof may be 1 or more).

In the present invention, the type of the target nucleic acid on which the first generation-type Staple nucleic acid forms a guanine quadruplex structure may be RNA or DNA. The type of the nucleic acid of a target nucleic acid is determined by a purpose to be achieved with the Staple nucleic acid.

For the first generation-type Staple nucleic acid of the present invention to form a guanine quadruplex structure in cells, the oligonucleotide is required to have both
- an ability to recognize the sequence of a target nucleic acid; and
- an ability to induce formation of a guanine quadruplex structure, at the same time.

The ability of the first generation-type Staple nucleic acid to recognize the sequence of a target nucleic acid is achieved by ensuring that an oligonucleotide which is a first generation-type Staple nucleic acid comprises a first nucleotide sequence and a second nucleotide sequence which hybridize with a nucleotide sequence adjacent to (i.e., on the 5' side or 3' side of) guanine repeat sequences on the target nucleic acid.

In the present invention, the sequences of the first nucleotide sequence and the second nucleotide sequence in the first generation-type Staple nucleic acid vary depending on the sequence of the target nucleic acid. It is possible to select two sequence portions on the target nucleic acid so that the spatial distance between four guanine repeat sequences on the target nucleic acid can be decreased, and to determine the first nucleotide sequence and the second nucleotide sequence so as to hybridize with the two sequence portions.

The first nucleotide sequence and the second nucleotide sequence of the first generation-type Staple nucleic acid can be appropriately designed. For example, but not limited to, an example of the first generation-type Staple nucleic acid of the invention is such that a nucleotide sequence on the downstream side (3' side) of one of guanine repeat sequences on the target nucleic acid (e.g., a region of 13 to 20 bases long starting from a base immediately downstream side (3' side) within 20 bases (i.e., the 1st to 20th bases on the downstream side of the guanine repeat sequence)), or a nucleotide sequence starting from a base immediately upstream side (5' side) within 20 bases of the guanine repeat sequence (i.e., the 1st to 20th bases on the downstream side of the guanine repeat sequence) on the target nucleic acid (e.g., a region of 13 to 20 bases long on the upstream side (5' side), and the like are selected, and the first nucleotide sequence and the second nucleotide sequence can be designed so as to hybridize those regions.

The distance from the guanine repeat sequence to the nucleotide sequence region on the target nucleic acid with which the first nucleotide sequence and the second nucleotide sequence hybridize (the number of nucleotides between the nucleotide at the most proximal end with respect to the guanine repeat sequence of the first nucleotide sequence or the second nucleotide sequence and G of the guanine repeat sequence) can be appropriately determined by calculation related to thermodynamic stability, and the distance (the number of nucleotides) is not limited. From studies by the present inventors, a guanine quadruplex structure has been confirmed to be formed even when the distance is long enough to sandwich 20 nucleotides. Therefore, the starting point of the region with which the Staple nucleic acid hybridizes can preferably be selected as a base within 20 bases from the immediately downstream side (3' side) or the immediately upstream side (5' side) of the guanine repeat sequence (i.e., the 1st to 20th bases on the downstream side or the upstream side of the guanine repeat sequence), more preferably a base within 15 bases from the immediately downstream side (3' side) or the immediately upstream side (5' side) of the guanine repeat sequence (i.e., the 1st to 15th bases on the downstream side or the upstream side of the guanine repeat sequence), more preferably a base within 10 bases from the immediately downstream side (3' side) or the immediately upstream side (5' side) of the guanine repeat sequence (i.e., the 1st to 10th bases on the downstream side or the upstream side of the guanine repeat sequence), more preferably a base within 5 bases from the immediately downstream side (3' side) or the immediately upstream side (5' side) of the guanine repeat sequence (i.e., the 1st to 5th bases on the downstream side or the upstream side of the guanine repeat sequence), and more preferably a base within 3 bases from the immediately downstream side (3' side) or the immediately upstream side (5' side) of the guanine repeat sequence (i.e., the 1st to 3rd bases on the downstream side or the upstream side of the guanine repeat sequence).

The first generation-type Staple nucleic acid may be composed of DNA, RNA, a modified nucleic acid, or a combination thereof. The first generation-type Staple nucleic acid of the present invention is formed by directly linking the first nucleotide sequence and the second nucleotide sequence, or by indirectly linking them via a linker. In the present invention, a type of a nucleic acid can be selected as appropriately for each of the first nucleotide sequence, the second nucleotide sequence, and an optionally contained linker.

Here, in the first generation-type Staple nucleic acid, the linker connecting between the first nucleotide sequence and the second nucleotide sequence refers to a linker of a nucleic acid having any number of bases between the first nucleotide sequence and the second nucleotide sequence. The linker is required to have a length such that, upon binding of the first nucleotide sequence with the target nucleic acid and binding of the second nucleotide sequence with the target nucleic acid, the first generation-type Staple nucleic acid can change the conformation of the target nucleic acid, whereby decreasing the spatial distance between the four guanine repeat sequences on the target nucleic acid.

The first generation-type Staple nucleic acid is an oligonucleotide which is formed by directly linking, or indirectly linking via a linker the first nucleotide sequence and the second nucleotide sequence. The overall length of the Staple nucleic acid varies depending on the nucleotide sequence of the target nucleic acid, may be any length designed based on criteria generally known in the art, such as a GC content and a Tm value, and may be a larger number of nucleotides in length depending on the design. As an example, the overall length of the Staple nucleic acid may be 26 to 40 nucleotides in length, but is not limited thereto. The length of the region where the Staple nucleic acid hybridizes with the target nucleic acid is not limited, and the length of either the region on one end side (e.g., 3' side) and that of the region on the other end side (e.g., 5' side) may be the same or different.

The hybridization reaction between the first generation-type Staple nucleic acid as designed above and the target nucleic acid can be carried out either *in vivo* or *in vitro,* and the hybridization reaction can be carried out by, for example,
- mixing a Staple nucleic acid in a solution containing a target nucleic acid, followed by annealing;
- simply mixing a Staple nucleic acid in a solution containing a target nucleic acid; or
- introducing a short hairpin expression vector and expressing the Staple nucleic acid from inside the cell.
The hybridization reaction between the target nucleic acid and the Staple nucleic acid can be carried out at a reaction temperature at, for example, 24°C to 55°C for, for example, 1 minute to 2 days.

The ability of the first generation-type Staple nucleic acid to form a guanine quadruplex structure is achieved by presence of four guanine repeat sequences on the target nucleic acid, by change of the conformation of the target nucleic acid via the Staple nucleic acid hybridized with the target nucleic acid to decrease the spatial distance between the four guanine repeat sequences on the target nucleic acid, and by, as a result, induction of a guanine quadruplex structure composed of the four guanine repeat sequences on the target nucleic acid.

The reaction of the guanine quadruplex structure formation using the first generation-type Staple nucleic acid of the present invention can be carried out by, for example, inducing a hybridization reaction between the target nucleic acid and the Staple nucleic acid, and allowing the reaction product to stand. The reaction of the guanine quadruplex structure formation may be performed at the same temperature and same time as the hybridization reaction of the target nucleic acid and the Staple nucleic acid, and may be carried out at a reaction temperature of, for example, 24°C to 55°C for, for example, 1 minute to 2 days. The formation of the guanine quadruplex structure can be confirmed by, for example, an *in vitro* translation assay, a fluorescence signal measurement method by reacting with thioflavin T, a stop assay method, or the like.

An example of a specific structure of the first generation-type Staple nucleic acid is shown in Chem. 1 below. The oligonucleotide designated as "Short nucleic acid" in Chem. 1 means a Staple nucleic acid, and introduction of the oligonucleotide brings four guanine repeat sequences on the target nucleic acid into close proximity to induce formation of a guanine quadruplex structure.

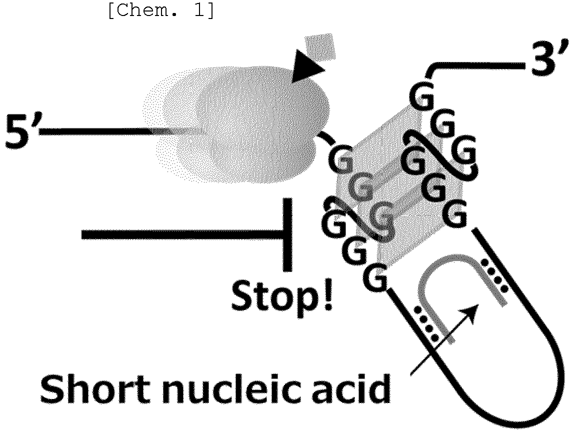

### Structure of second generation-type Staple nucleic acid

The second generation-type Staple nucleic acid in the present invention refers to a Staple nucleic acid which supplies a part of four guanine repeat sequences constituting a guanine quadruplex structure by an oligonucleotide. That is, the second generation-type Staple nucleic acid is structurally characterized in that the Staple nucleic acid itself contains guanine repeat sequence(s).

The second generation-type Staple nucleic acid in the present invention refers to a G supply-type oligonucleotide comprising: a first nucleotide sequence and a second nucleotide sequence, which target a nucleotide sequence portion containing one to three guanine repeat sequences on a target nucleic acid, and each hybridize with a nucleotide sequence adjacent to (i.e., on the 5' side or 3' side of) any of the guanine repeat sequences; and three to one guanine repeat sequences depending on the number of guanine repeat sequences on the target nucleic acid,
wherein the G supply-type oligonucleotide changes the conformation of the target nucleic acid through hybridization of the first nucleotide sequence and the second nucleotide sequence with the target nucleic acid, decreases a spatial distance between a total of four guanine repeat sequences consisting of the guanine repeat sequences on the target nucleic acid and the guanine repeat sequences on the G supply-type oligonucleotide, and forms a guanine quadruplex structure composed of the four guanine repeat sequences.

Here, the phrase "containing one to three guanine repeat sequences on the target nucleic acid" means that at least one guanine repeat sequence for forming a guanine quadruplex structure is present on the target nucleic acid and may contain four or more guanine repeat sequences on the target nucleic acid. When four or more guanine repeat sequences are present on the target nucleic acid, it means that a guanine quadruplex structure is formed by one to three of the guanine repeat sequences. In addition, the term "the number of guanine repeat sequences on the target nucleic acid" means the number of guanine repeat sequences on the target nucleic acid, which are used to form a guanine quadruplex structure using the second generation-type Staple nucleic acid of the present invention. For example, even when four or more guanine repeat sequences are present on the target nucleic acid, and when two of the guanine repeat sequences on the target nucleic acid and two guanine repeat sequences supplied by the G supply-type oligonucleotide form a guanine quadruplex structure, "the number of guanine repeat sequences on the target nucleic acid" is intended to be 2.

As described above, in general, the guanine quadruplex structure in cells is a stable nucleic acid higher-order structure formed when four guanine repeat sequences each having 3 or more bases are present in close spatial arrangement of less than 7 bases. The present inventors of the present invention have found that even when one to three guanine repeat sequences are present at distant locations with spacing of 7 or more bases on the target nucleic acid, by using a G supply-type oligonucleotide, appropriately designed according to the present invention, containing: a first nucleotide sequence and a second nucleotide sequence; and three to one guanine repeat sequences depending on the number of guanine repeat sequences, a total of four guanine repeat sequences consisting of one to three guanine repeat sequences present on the target nucleic acid and three to one guanine repeat sequences present on the G supply-type oligonucleotide can be artificially brought into spatial proximity to induce an artificial guanine quadruplex structure on the target nucleic acid.

As used herein, the guanine repeat sequence in a target nucleic acid to which the second generation-type Staple nucleic acid binds refers to a consecutive sequence containing 3 or more guanine bases present on the target nucleic acid, or a consecutive sequence which also contains 3 or more guanine bases present on the Staple nucleic acid. Examples of the specific guanine repeat sequence include guanine repeat sequences that can be elements for forming guanine quadruplex structures reported in the literature, such as GGG, and GNGG (N represents any nucleotide).

In the present invention, the type of the target nucleic acid on which the second generation-type Staple nucleic acid forms a guanine quadruplex structure may be RNA or DNA as in the case of the first generation-type Staple nucleic acid. The type of the nucleic acid of a target nucleic acid is determined by a purpose to be achieved with the Staple nucleic acid.

For the second generation-type Staple nucleic acid of the present invention to form a guanine quadruplex structure in cells, the oligonucleotide is required to have, at the same time
- an ability to recognize the sequence of a target nucleic acid;
- an ability to supply guanine repeat sequences, depending on the number of guanine repeat sequences present on the target nucleic acid, so that the total number of guanine repeat sequences becomes 4 (note that when four or more guanine repeat sequences are present on the target nucleic acid, one to three of the guanine repeat sequences are used); and
- an ability to induce the formation of a guanine quadruplex structure.

The ability of the second generation-type Staple nucleic acid to recognize the sequence of a target nucleic acid is achieved by ensuring that an oligonucleotide which is a second generation-type Staple nucleic acid comprises a first nucleotide sequence and a second nucleotide sequence which hybridize with a nucleotide sequence adjacent to (i.e., on the 5' side or 3' side of) guanine repeat sequences on the target nucleic acid.

In the present invention, the sequences of the first nucleotide sequence and the second nucleotide sequence in the second generation-type Staple nucleic acid vary depending on the sequence of the target nucleic acid. It is possible to select two sequence portions on the target nucleic acid so that the spatial distance between a total of four guanine repeat sequences consisting of one to three guanine repeat sequences on the target nucleic acid and three to one guanine repeat sequences on the second generation-type Staple nucleic acid can be decreased, and to determine the first nucleotide sequence and the second nucleotide sequence so as to hybridize with the two sequence portions.

The first nucleotide sequence and the second nucleotide sequence of the second generation-type Staple nucleic acid can be appropriately designed. For example, the first nucleotide sequence and the second nucleotide sequence of the Staple nucleic acid may be selected so as to hybridize with
- a nucleotide sequence portion adjacent to (i.e., on the 5' side or 3' side of) the 3'-most guanine repeat sequence of the guanine repeat sequences on the target nucleic acid; and
- a nucleotide sequence portion adjacent to (i.e., on the 5' side or 3' side of) the 5'-most guanine repeat sequence of the guanine repeat sequences on the target nucleic acid.

For example, an example of the second generation-type Staple nucleic acid of the invention is such that a region of 13 to 20 bases long on the immediately downstream side (3' side) of one of guanine repeat sequences on the target nucleic acid, and a region of 13 to 20 bases long on the upstream side (5' side) of the guanine repeat sequence on the target nucleic acid are selected, and the first nucleotide sequence and the second nucleotide sequence can be designed so as to hybridize with those regions.

The first nucleotide sequence and the second nucleotide sequence of the second generation-type Staple nucleic acid of the present invention can be appropriately designed according to the intended use thereof. For example, the first nucleotide sequence and the second nucleotide sequence can be appropriately designed so as to have the following characteristics (but are not limited to those):
- at least one of the first nucleotide sequence and the second nucleotide sequence hybridizes with a nucleotide sequence portion adjacent to a guanine repeat sequence present before a stop codon on the target nucleic acid;
- at least one of the first nucleotide sequence and the second nucleotide sequence hybridizes with a nucleotide sequence portion adjacent to a guanine repeat sequence present in a 5' untranslated region on the target nucleic acid;
- the first nucleotide sequence and the second nucleotide sequence both hybridize with a nucleotide sequence portion adjacent to a guanine repeat sequence present in a 5' untranslated region on the target nucleic acid; or
- the first nucleotide sequence and the second nucleotide sequence both hybridize with a nucleotide sequence portion adjacent to a guanine repeat sequence present in a 3' untranslated region.

However, in the case of use for other purpose, the nucleotide sequences are appropriately designed in accordance with the other intended use.

The distance from the guanine repeat sequence to the nucleotide sequence region on the target nucleic acid with which the first nucleotide sequence and the second nucleotide sequence hybridize (the number of nucleotides between the nucleotide at the most proximal end with respect to the guanine repeat sequence of the first nucleotide sequence or the second nucleotide sequence and G of the guanine repeat sequence) can be appropriately determined by calculation related to thermodynamic stability, and the distance (the number of nucleotides) is not limited. From studies by the present inventors, a guanine quadruplex structure has been confirmed to be formed even when the distance is long enough to sandwich 20 nucleotides.

The second generation-type Staple nucleic acid may be composed of DNA, RNA, a modified nucleic acid, or a combination thereof. The second generation-type Staple nucleic acid of the present invention is formed by directly linking the first nucleotide sequence, the second nucleotide sequence, three to one guanine repeat sequences, or by indirectly linking them via a linker. In the present invention, a type of a nucleic acid can be selected as appropriately for each of the first nucleotide sequence, the second nucleotide sequence, three to one guanine repeat sequences, and a linker that is optionally contained.

Here, in the second generation-type Staple nucleic acid, the linker connecting between the first nucleotide sequence and the second nucleotide sequence refers to linkers of a nucleic acid present between the first nucleotide sequence and the second nucleotide sequence, between the first nucleotide sequence and/or the second nucleotide sequence and the guanine repeat sequence on the Staple nucleic acid, or between the guanine repeat sequences on the Staple nucleic acid, which have any number of bases; which are other than the first nucleotide sequence, the second nucleotide sequence and the guanine repeat sequence. The linker is required to have a length such that, upon binding between the first nucleotide sequence with the target nucleic acid and binding between the second nucleotide sequence with the target nucleic acid, the second generation-type Staple nucleic acid cay change the conformation of the target nucleic acid, whereby decreasing the spatial distance between a total of four guanine repeat sequences consisting of guanine repeat sequences on the target nucleic acid and guanine repeat sequences on the Staple nucleic acid.

The second generation-type Staple nucleic acid is an oligonucleotide which is formed by directly linking, or indirectly linking via linker, the first nucleotide sequence, the second nucleotide sequence and three to one guanine repeat sequences. The overall length of the Staple nucleic acid varies depending on the nucleotide sequence of the target nucleic acid, may be any length designed based on criteria generally known in the art, such as a GC content and a Tm value. As an example, the overall length of the Staple nucleic acid may be 26 to 40 nucleotides in length, but is not limited thereto. The length of the region where the Staple nucleic acid hybridizes with the target nucleic acid is not limited, and the length of either the region on one end side (e.g., 3' side) and that of the region on the other end side (e.g., 5' side) may be the same or different.

The hybridization reaction between the second generation-type Staple nucleic acid as designed above and the target nucleic acid can be carried out either *in vivo* or *in vitro,* as in the case of the first generation-type Staple nucleic acid, and the hybridization reaction can be carried out by, for example,
- mixing a Staple nucleic acid in a solution containing a target nucleic acid, followed by annealing;
- simply mixing a Staple nucleic acid in a solution containing a target nucleic acid; or
- introducing a short hairpin expression vector and expressing the Staple nucleic acid from inside the cell.
The hybridization reaction between the target nucleic acid and the Staple nucleic acid may be carried out at a reaction temperature at, for example, 24°C to 55°C for, for example, 1 minute to 2 days.

In one embodiment of the present invention, the second generation-type Staple nucleic acid is characterized by having an ability to supply, depending on the number of guanine repeat sequences present on the target nucleic acid, guanine repeat sequences so that the total number of guanine repeat sequences becomes 4. That is, regardless of the number of guanine repeat sequences present on the target nucleic acid, the Staple nucleic acid can supply three guanine repeat sequences when any one of guanine repeat sequences present on the target nucleic acid is used to form a guanine quadruplex structure, the Staple nucleic acid can supply two guanine repeat sequences when any two of guanine repeat sequences present on the target nucleic acid are used to form a guanine quadruplex structure, and the Staple nucleic acid can supply one guanine repeat sequence when any three of guanine repeat sequences present on the target nucleic acid are used to form a guanine quadruplex structure.

Since the second generation-type Staple nucleic acid as described above can supply guanine repeat sequence(s) for forming a guanine quadruplex structure, the second generation-type Staple nucleic acid can also be called a G supply-type oligonucleotide or a G supply-type Staple nucleic acid in the context that it is an oligonucleotide which supplies guanine repeat sequences.

The ability of the second generation-type Staple nucleic acid to form a guanine quadruplex structure is achieved by presence of a total of four guanine repeat sequences consisting of guanine repeat sequences present on the target nucleic acid and guanine repeat sequences present on the second generation-type Staple nucleic acid, by change of the conformation of the target nucleic acid via the Staple nucleic acid hybridized with the target nucleic acid to decrease the spatial distance between a total of four guanine repeat sequences on the target nucleic acid and the second generation-type Staple nucleic acid, and by, as a result, induction of a guanine quadruplex structure composed of a total of four guanine repeat sequences on the target nucleic acid and the second generation-type Staple nucleic acid.

The reaction of the guanine quadruplex structure formation using the second generation-type Staple nucleic acid of the present invention can be carried out by, for example, inducing a hybridization reaction between the target nucleic acid and the Staple nucleic acid, and allowing the reaction product to stand. The reaction of the guanine quadruplex structure formation may be performed at the same temperature and same time as the hybridization reaction of the target nucleic acid and the Staple nucleic acid, and may be carried out at a reaction temperature of, for example, 24°C to 55°C for, for example, 1 minute to 2 days. The formation of the guanine quadruplex structure can be confirmed by, for example, an *in vitro* translation assay, a fluorescence signal measurement method by reacting with thioflavin T, a stop assay method, or the like.

An example of a specific structure is shown in Chem. 2 below. In Chem. 2, pattern A shows a case where three guanine repeat sequences are supplied by an oligonucleotide, pattern B shows a case where two guanine repeat sequences are supplied by an oligonucleotide, and pattern C shows a case where one guanine repeat sequence is supplied by an oligonucleotide. Introduction of the oligonucleotide decreases a spatial distance between a total of four guanine repeat sequences consisting of guanine repeat sequences on the target nucleic acid and guanine repeat sequences on the G supply-type oligonucleotide, to induce formation of a guanine quadruplex structure by these four guanine repeat sequences.

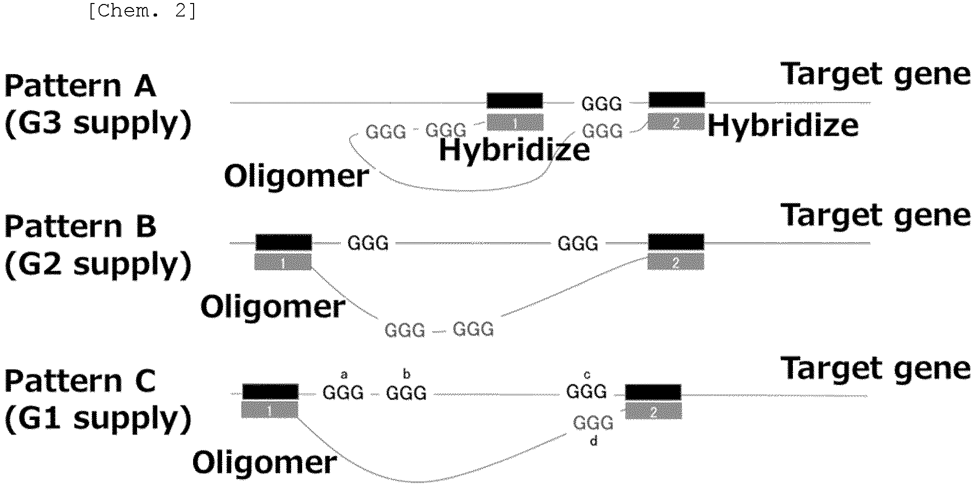

### Method for producing second generation-type Staple nucleic acid

The present invention can also provide a method for producing the second generation-type Staple nucleic acid described above (G supply-type oligonucleotide).

Specifically, it is possible to provide a method for producing a G supply-type oligonucleotide comprising: a first nucleotide sequence and a second nucleotide sequence, which target a nucleotide sequence portion containing one to three guanine repeat sequences on a target nucleic acid, and each hybridize with a nucleotide sequence adjacent to (i.e., on the 5' side or 3' side of) the guanine repeat sequences; and three to one guanine repeat sequences depending on the number of guanine repeat sequences on the target nucleic acid, the method comprising designing and synthesizing the G supply-type oligonucleotide such that the first nucleotide sequence and the second nucleotide sequence of the G supply-type oligonucleotide hybridize with the target nucleic acid, and the G supply-type oligonucleotide is folded so as to decrease a spatial distance between a total of four guanine repeat sequences consisting of guanine repeat sequences on the target nucleic acid and guanine repeat sequences on the G supply-type oligonucleotide.

In production of the second generation-type Staple nucleic acid of the present invention, it is important to appropriately design the structure of the Staple nucleic acid. Specifically, it is important to appropriately design the first nucleotide sequence and the second nucleotide sequence to be hybridized with the target nucleic acid, the number of guanine repeat sequences supplied, and one or more optional linkers which link between the sequence portions. In designing sequences constituting the Staple nucleic acid, the sequences for forming the Staple nucleic acid are preferably designed so as to reflect the characteristics of the second generation-type Staple nucleic acid. Once the structure of the Staple nucleic acid can be designed, the Staple nucleic acid itself can be synthesized by a common nucleic acid synthesis method.

### Functions of nucleic acid oligonucleotide

Both the first generation-type Staple nucleic acid and the second generation-type Staple nucleic acid of the present invention can exhibit the same functions as a result of formation of a guanine quadruplex structure. In other words, the functions exhibited by the first generation-type Staple nucleic acid can also be exhibited by the second generation-type Staple nucleic acid, and functions exhibited by the second generation-type Staple nucleic acid can also be exhibited by the first generation-type Staple nucleic acid. This is because the functions of the Staple nucleic acid of the present invention result from the formation of a guanine quadruplex structure, and the properties of guanine quadruplex structures formed are similar in both the first generation-type Staple nucleic acid and the second generation-type Staple nucleic acid.

### (1) Downregulation of protein expression

In the present invention, protein expression from the target nucleic acid can be downregulated as a result of formation of a guanine quadruplex structure on the target nucleic acid.

A specific example of a method for downregulating protein expression is a method of downregulating protein expression resulting from suppression of protein translation reaction, as a result of formation of a guanine quadruplex structure to prevent ribosomes from binding to the target nucleic acid (mRNA) or to stop 5' to 3' movement of ribosomes bound to the target nucleic acid (mRNA) at the location of the guanine quadruplex structure. It is possible to design the sequence of the Staple nucleic acid of the present invention so as to form a guanine quadruplex structure in, for example, a 5' UTR region or an open reading frame region on the target nucleic acid, to exhibit the function.

As described in Patent Literature 1 as an example of the first generation-type Staple nucleic acid, an oligonucleotide having the above-mentioned structure can suppress protein translation reaction by forming a guanine quadruplex structure via involvement of guanine repeat sequences located in a 5' untranslated region (5' UTR) of the target nucleic acid, and thus resulting in an enzyme-independent protein translation suppression system (Chem. 3, upper left picture). This is because even when ribosomes bind to the 5' UTR of the target nucleic acid, the ribosomes are prevented from moving to the 3' side of the target nucleic acid by the guanine quadruplex structure, and protein translation stops.

Another example of the method for suppressing expression of protein is as follows. As a result of formation of a guanine quadruplex structure, a reverse transcriptase cannot bind to the target nucleic acid (RNA), or movement of the reverse transcriptase bound to the target nucleic acid (RNA) in the direction of 3'→5' stops at the location of the guanine quadruplex structure, and protein expression reaction is thus downregulated.

Accordingly, the present invention can provide a method for downregulating protein expression from a target nucleic acid, using a G supply-type oligonucleotide comprising: a first nucleotide sequence and a second nucleotide sequence which target a nucleotide sequence portion containing one to three guanine repeat sequences on a target nucleic acid, and each hybridize with a nucleotide sequence adjacent to (i.e., on the 5' side or 3' side of) the guanine repeat sequences; and three to one guanine repeat sequences depending on the number of guanine repeat sequences on the target nucleic acid, to change the conformation of the target nucleic acid through hybridization of the first nucleotide sequence and the second nucleotide sequence with the target nucleic acid, to decrease a spatial distance between a total of four guanine repeat sequences consisting of the guanine repeat sequences on the target nucleic acid and the guanine repeat sequences on the G supply-type oligonucleotide, to form a guanine quadruplex structure composed of the four guanine repeat sequences, by the G supply-type oligonucleotide.

In the present invention, it has been demonstrated that even in the case of a guanine quadruplex structure formed by second generation oligonucleotides, the protein expression downregulating function shown here is similarly exhibited even when the guanine quadruplex structure to be formed by the oligonucleotides is formed in relation to guanine repeat sequences present in the 5' untranslated region (5' UTR) of the target nucleic acid.

### (2) Stabilization of target nucleic acid

The other of the cases shown in Chem. 3 is a case where, as a result of formation of a guanine quadruplex structure, movement of a nucleolytic enzyme bound to mRNA, such as exonuclease, in the 5' direction stops at the location of the guanine quadruplex structure, and target nucleic acid decomposition reaction is suppressed. When the guanine quadruplex structure is formed by a total of four guanine repeat sequences including guanine repeat sequences present in the 3' untranslated region (3' UTR) of the target nucleic acid, the exonuclease is prevented from moving to the 5' side of the target nucleic acid by the guanine quadruplex structure, and the decomposition action of the 3'→5' exonuclease on the target nucleic acid is suppressed (Chem. 3, upper right picture). For exhibiting such a function, the sequence of the Staple nucleic acid of the present invention is designed so as to form a guanine quadruplex structure in a 3' UTR region on the target nucleic acid when the target nucleic acid is mRNA.

Since the decomposition action is stopped as described above, the target nucleic acid is stabilized, and the amount of translation of protein from the target nucleic acid increases.

Accordingly, the present invention can provide a method for stabilizing a target nucleic acid, using an oligonucleotide comprising: a first nucleotide sequence and a second nucleotide sequence which target a nucleotide sequence portion containing one to four guanine repeat sequences on a target nucleic acid, and each hybridize with a nucleotide sequence adjacent to (i.e., on the 5' side or 3' side of) the guanine repeat sequences; and three to zero guanine repeat sequences depending on the number of guanine repeat sequences on the target nucleic acid, wherein the oligonucleotide inhibits binding of a nucleolytic enzyme to the target nucleic acid or inhibits functions of the nucleolytic enzyme by changing the conformation of the target nucleic acid through hybridization of the first nucleotide sequence and the second nucleotide sequence with the target nucleic acid, decreasing a spatial distance between a total of four guanine repeat sequences consisting of guanine repeat sequences on the target nucleic acid and guanine repeat sequences on the oligonucleotide, to form a guanine quadruplex structure composed of the four guanine repeat sequences.

Either in the case of a guanine quadruplex structure formed by first generation oligonucleotides or in the case of a guanine quadruplex structure formed by second generation oligonucleotides, the function of suppressing target nucleic acid decomposition is similarly exhibited when the guanine quadruplex structure to be formed by the oligonucleotides is formed in relation to guanine repeat sequences present in the 3' untranslated region (3' UTR) of the target nucleic acid.

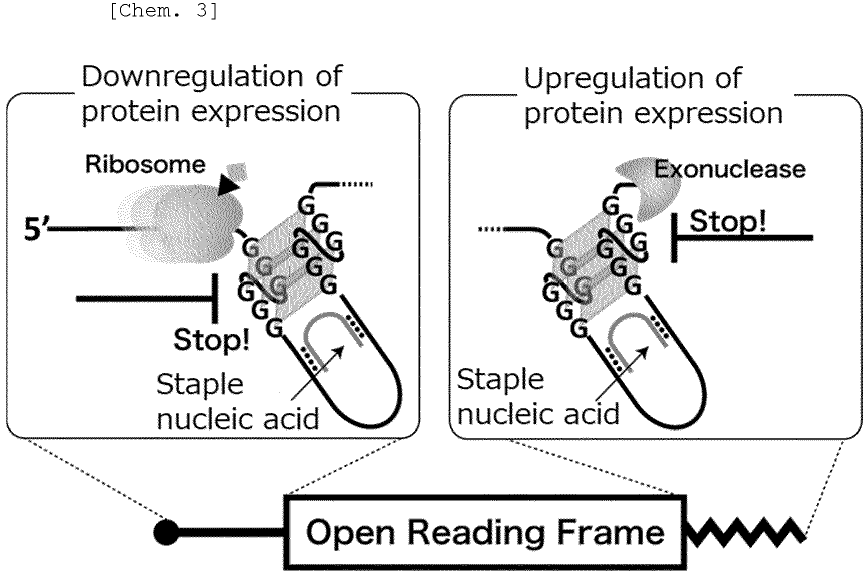

### (3) Ribosomal shunting and modified translation of sub-sequence in single target nucleic acid

When the guanine quadruplex structure to be formed by the oligonucleotide of the present invention (including the first generation oligonucleotide and the second generation oligonucleotide) is formed in an exon region of the target nucleic acid, a loop is thus formed by the guanine quadruplex structure, ribosomes bound to the target nucleic acid switch over the target nucleic acid (i.e., performing ribosomal shunting) at the guanine quadruplex structure portion when moving in the 3' direction, and a sub-sequence-modified protein of the target nucleic acid can be translated.

Accordingly, the present invention can provide a method for producing a modified protein from a target nucleic acid using an oligonucleotide comprising: a first nucleotide sequence and a second nucleotide sequence, which target a nucleotide sequence portion containing one to four guanine repeat sequences on a target nucleic acid, and each hybridize with a nucleotide sequence adjacent to (i.e., on the 5' side or 3' side of) the guanine repeat sequences; and three to zero guanine repeat sequences depending on the number of guanine repeat sequences on the target nucleic acid, wherein the oligonucleotide forms a loop portion on a part of the target nucleic acid, by changing the conformation of the target nucleic acid through hybridization of the first nucleotide sequence and the second nucleotide sequence with the target nucleic acid, decreasing a spatial distance between a total of four guanine repeat sequences consisting of guanine repeat sequences on the target nucleic acid and guanine repeat sequences on the oligonucleotide, to form a guanine quadruplex structure composed of the four guanine repeat sequences, and ribosomes shunt over the loop portion.

Either in the case of a guanine quadruplex structure formed by first generation oligonucleotides or in the case of a guanine quadruplex structure formed by second generation oligonucleotides, the function of modifying and translating a sub-sequence of the target nucleic acid is similarly exhibited when the guanine quadruplex structure to be formed by the oligonucleotides is formed in relation to guanine repeat sequences present in the exon region of the target nucleic acid.

### (4) Ribosomal shunting and fusion translation of sub-sequences between two types of target nucleic acids

The oligonucleotide of the present invention (including the first generation oligonucleotide and the second generation oligonucleotide) can also form a guanine quadruplex structure between two types of target nucleic acids (herein, sometimes referred to as a first target nucleic acid and a second target nucleic acid), and the two types of target nucleic acids can be substantially connected. When, if the nucleic acids connected as described above are mRNA, the reaction of ribosomes proceeds with respect to the mRNA, a reaction called a ribosomal shunt proceeds because helicase activity does not work and a stable nucleic acid higher-order structure is bypassed. It has been reported that the phenomenon of the ribosomal shunt occurs only when a very long Stem structure is constructed. On the other hand, the guanine quadruplex structure formed by the oligonucleotide of the present invention is a very stable nucleic acid higher-order structure, and therefore can induce a ribosomal shunt between two types of target nucleic acids without the need for the long Stem structure.

When a guanine quadruplex structure is formed at the exon region in each of the two types of target nucleic acids, ribosomes bound to the first target nucleic acid switch over to the second target nucleic acid from the first target nucleic acid (i.e., performing ribosomal shunting) at the guanine quadruplex structure portion formed in the exon when moving in the 3' direction, and a translated product fused with a sub-sequence of the first target nucleic acid and a sub-sequence of the second target nucleic acid can be translated (Chem. 4).

Accordingly, the present invention can provide a method for producing a fused protein of a translated product fused with a sub-sequence of a first target nucleic acid and a sub-sequence of a second target nucleic acid, using an oligonucleotide comprising: a first nucleotide sequence which targets a nucleotide sequence portion containing one to two guanine repeat sequences on the first target nucleic acid, and hybridizes with a nucleotide sequence adjacent to (i.e., on the 5' side or 3' side of) the guanine repeat sequences; a second nucleotide sequence which is directed to a nucleotide sequence portion containing one to two guanine repeat sequences on the second target nucleic acid, and hybridizes with a nucleotide sequence adjacent to (i.e., on the 5' side or 3' side of) the guanine repeat sequences; and two to zero guanine repeat sequences depending on the number of the guanine repeat sequences on the first target nucleic acid and the number of the guanine repeat sequences on the second target nucleic acid, wherein the oligonucleotide hybridizes with the first target nucleic acid through its first nucleotide sequence and with the second target nucleic acid through its second nucleotide sequence, changes the conformation of the target nucleic acids, decreases a spatial distance between a total of four guanine repeat sequences consisting of one to two guanine repeat sequences on the first target nucleic acid, one to two guanine repeat sequences on the second target nucleic acid and two to zero guanine repeat sequences on the oligonucleotide, to form a guanine quadruplex structure composed of the four guanine repeat sequences, and ribosomes shunt between the first target nucleic acid and the second target nucleic acid.

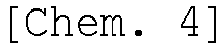

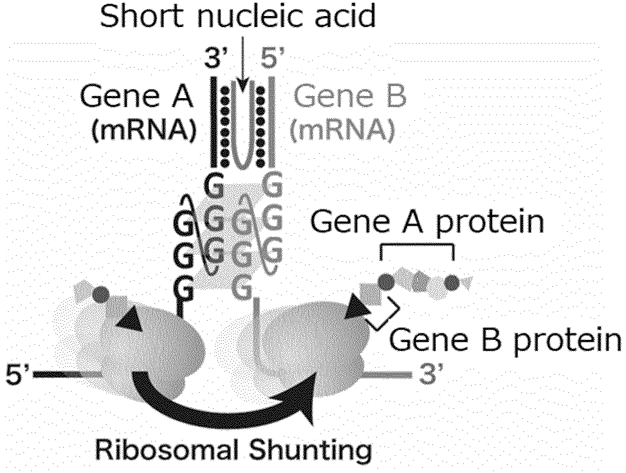

### Uses of Staple nucleic acid of invention

Since the Staple nucleic acid (oligonucleotide) of the present invention has the functions described in the section "Functions of nucleic acid oligonucleotide" above, the present invention can use the functions to provide a pharmaceutical composition comprising the Staple nucleic acid of the present invention.

The pharmaceutical composition comprising the Staple nucleic acid of the present invention may be provided in such a form that the Staple nucleic acid prepared outside the body is administered, or in such a form that a vector which produces the Staple nucleic acid is administered to a living organism to produce the Staple nucleic acid *in vivo.* Therefore, the pharmaceutical composition of the present invention can be prepared by formulating a therapeutically effective amount of the Staple nucleic acid of the present invention alone or together with a pharmaceutically acceptable carrier (additive), excipient and/or diluent, or by formulating a vector, which enables a therapeutically effective amount of the Staple nucleic acid of the present invention to be produced *in vivo,* alone or together with a pharmaceutically acceptable carrier (additive), excipient and/or diluent.

The term "therapeutically effective amount" as used herein refers to an amount of the Staple nucleic acid of the present invention, which is effective for obtaining a therapeutic effect based on a desirable primary action, regardless of the presence or absence of a side effect.

The term "pharmaceutically acceptable carrier" as used herein refers to a carrier commonly used in the art, which means a carrier that does not cause excessive toxicity, stimulus or allergy reaction, or other problems or complications, and is suitable for application to human and animal tissues. Examples of the carrier include, but are not limited to, pharmaceutically acceptable materials, compositions or vehicles that are involved in delivery or transportation of an intended compound from an organ or a body part to another organ or body part, for example, liquid or solid fillers, diluents, excipients, production aids (e.g., lubricants, talc, magnesium, calcium stearate, zinc stearate and stearic acid), and solvents including materials.

The pharmaceutical composition comprising the Staple nucleic acid of the present invention can be used in combination with another component. The other component is not limited. Any component may be used in combination.

Since the Staple nucleic acid (oligonucleotide) of the present has the functions described in the section "Functions of nucleic acid oligonucleotide" above, it is also possible to provide a kit for realizing the functions, comprising the Staple nucleic acid of the present invention. Specifically, it is possible to provide
- a kit for downregulating protein expression, comprising a Staple nucleic acid, which is intended to realize the function of "(1) Downregulation of protein expression" above;
- a kit for stabilizing a target nucleic acid, comprising a Staple nucleic acid, which is intended to realize the function of "(2) Stabilization of target nucleic acid" above;
- a kit for producing a sub-sequence-modified protein of a target nucleic acid, comprising a Staple nucleic acid, which is intended to realize the function of "(3) Ribosomal shunting and modified translation of sub-sequence in single target nucleic acid" above;
- and a kit for producing a sub-sequence-fused protein of a target nucleic acid, comprising a Staple nucleic acid, which is intended to realize the function of "(4) Ribosomal shunting and fusion translation of sub-sequences between two types of target nucleic acids" above. The kit contents can be broadened as the terms of the Staple nucleic acid of the present invention broaden.

For example, in a more specific embodiment, as the kit for downregulating protein expression, comprising a Staple nucleic acid, which is intended to realize the function of "(1) Downregulation of protein expression" above, it is possible to provide, for example, a kit for downregulating protein expression comprising a G supply-type oligonucleotide comprising: a first nucleotide sequence and a second nucleotide sequence, which target a nucleotide sequence portion containing one to three guanine repeat sequences on a target nucleic acid, and each hybridize with a nucleotide sequence adjacent to (i.e., on the 5' side or 3' side of) the guanine repeat sequences; and three to one guanine repeat sequences depending on the number of guanine repeat sequences on the target nucleic acid, wherein the G supply-type oligonucleotide downregulates protein expression from the target nucleic acid by changing the conformation of the target nucleic acid through hybridization of the first nucleotide sequence and the second nucleotide sequence with the target nucleic acid, decreasing a spatial distance between a total of four guanine repeat sequences consisting of guanine repeat sequences on the target nucleic acid and guanine repeat sequences on the G supply-type oligonucleotide, to form a guanine quadruplex structure composed of the four guanine repeat sequences.

Hereinafter, the present invention will be described in more detail by showing Examples. Examples shown below is in no way intended to limit the present invention.

### Examples

### Example 1: Design of second generation-type Staple nucleic acid

In this Example, a second generation-type Staple nucleic acid, which targets some mRNAs and supplies guanine repeat sequences from an oligonucleotide to form a guanine quadruplex structure, was created.
(1) Second generation-type Staple nucleic acid binding to 1+1 100nt RNA as target nucleic acid

In this experiment, a model nucleic acid RNA sequence (1+1 100nt RNA), in which a 100-base loop sequence contains one adjacent guanine repeat sequence on each ends of the loop sequence, was designed and used. The nucleotide sequence of 1+1 100nt RNA as a target nucleic acid is as follows.

### [Chem. 5]

### <1+1 100nt RNA>

5'_ACACAGGAAACAGCUAUGACCAUGAUUACGCCAAGUUUGCACGCCAGCCGUUCGACGA
UUUAAUACGACUCACUAUAGAUUAGCAUACGCUACUGCAGUGGGUAAAUGUCGACCUAG
AUUAAUGCAAUUCGUGUGAAGAUCCUUUUUGAUAAUCUCAUGACCAAAAUCCCUUAACG
UGAGUUCAUUUCCAGCACCCAAUUGAAGCUUUAAAUGGGUGCAUGCCAUGACUUGCAAG
CUAUUCAGCAGGUAUACAUAGUUGGAAAUCUCUGGAAGAUCCGCGCGUACCGAGUUCUA
AUUCACUGGCCGUCGUUUUACAACGUCGUGACUGGGAAAACCCUGGCGUUACCCAACUU
AAUCGCCUUGCAGCACAUCC_3' (SEQ ID NO: 1)

Boxed region: the guanine repeat sequence used in formation of guanine quadruplex structure
Underlined region: the region where G supply-type Staple nucleic acid is bound

This sequence has two guanine repeat sequences in the target nucleic acid, and can form a guanine quadruplex structure only when two guanine repeat sequences are supplied by the second generation-type Staple nucleic acid of the present invention.

For this target nucleic acid, the following oligonucleotides were designed and created. In the designing of the oligonucleotides, an attempt was made to design type I and type II oligonucleotides based on a positional relationship between a binding site of a first nucleotide sequence and a binding site of a second nucleotide sequence on the target nucleic acid and guanine repeat sequences on the target nucleic acid. That is, the type I oligonucleotide has two guanine repeat sequences located between the first nucleotide sequence and the second nucleotide sequence, whereas the type II oligonucleotide has two guanine repeat sequences located so as to sandwich the first nucleotide sequence and the second nucleotide sequence.

The following type I oligonucleotides were designed and synthesized:
(1-1) G supply-type Staple nucleic acid (for 1+1 100nt RNA, type I): G supply-type Staple nucleic acid of type I which binds to 1+1 100nt RNA as a target nucleic acid, and
(1-2) Staple_A (for 1+1 100nt RNA, type I): an oligonucleotide which was prepared by changing the guanine repeat sequence portions of the (1-1) oligonucleotide to adenine repeat sequences.

The oligonucleotides used herein were all purchased from Thermo Fisher Scientific, Inc. All the samples were purified by desalting before use.

### [Chem. 6]

### Staple nucleic acid of type I

(1-1) G supply-type Staple nucleic acid (for 1+1 100nt RNA, type I) ATTAATCTAGGTCGACATTTTGGGTGGGTTTTAAAGCTTCAATTGGGTG (SEQ ID NO: 2)
(1-2) Staple_A (for 1+1 100nt RNA, type I)
   ATTAATCTAGGTCGACATTTTAAATAAATTTTAAAGCTTCAATTGGGTG (SEQ ID NO: 3)
Boxed region: the guanine repeat sequence used in formation of guanine quadruplex structure
Underlined region: the region where G supply-type Staple nucleic acid is bound

The following type II oligonucleotides were designed and prepared:
(1-3) G supply-type Staple nucleic acid (type II): G supply-type Staple nucleic acid of type II which binds to 1+1 100nt RNA as a target nucleic acid, and
(1-4) Staple_A (type II): an oligonucleotide which was prepared by changing the guanine repeat sequence portions of the (1-3) oligonucleotide to adenine repeat sequences.

[Chem. 7]

### Staple nucleic acid of type II

(1-3) G supply-type Staple nucleic acid (for 1+1 100nt RNA, type I) GGGTTTTACAGCTGGATCTAATTAGTGGGTTAACTTCGAAATTTTGGG (SEQ ID NO: 4)
(1-4) Staple_A (for 1+1 100nt RNA, type II)
   AAATTTTACAGCTGGATCTAATTAGTGGGTTAACTTCGAAATTTTGGG (SEQ ID NO: 5)
Boxed region: the guanine repeat sequence used in formation of guanine quadruplex structure
Underlined region: the first nucleotide sequence and the second nucleotide sequence

These oligonucleotides were used to perform fluorescence measurement using Thioflavin T (ThT), known to bind to a guanine quadruplex structure and emit fluorescence, for evaluating the function of the G supply-type Staple nucleic acid to form a guanine quadruplex structure on the target nucleic acid.

The fluorescence measurement using Thioflavin T (ThT) was performed by fluorescence emission measurement for sample for an RNA sample of SEQ ID NO: 1 prepared under the following conditions. A mixed solution (total volume: 50 µl (micro liter)) of the RNA sample (2 µM (micro M)), KCl (100 mM), 20 mM Tris-HCl buffer (pH 7.6) and a G supply-type Staple nucleic acid (2.5 µM (micro M)) was heated at 90°C for 2 minutes, and cooled to 20°C at 1°C per minute. Thereafter, 10 µl (micro liter) of a 3 µM (micro M) ThT solution was added to the mixed solution at a final concentration of 0.5 µM (micro M) (Total volume: 60 µl (micro liter)), and the mixture was incubated at room temperature for 30 minutes. Using an FP-8500 spectrofluorometer (JASCO, Inc.), this sample was excited with light having a wavelength of 440 nm, and a fluorescence signal at 487 nm was measured. In the measurement, the measurement wavelength was 450 nm to 600 nm, the sweep rate was 200 nm/min, and the number of times of scanning was 3.

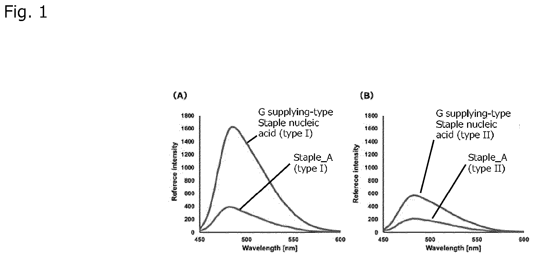

The results of the fluorescence measurement using the type I oligonucleotides (1-1) and (1-2) are shown in Figure 1(A). That is, a large fluorescence signal derived from ThT was exerted in the presence of the G supply-type Staple nucleic acid (1-1), whereas the fluorescence signal did not significantly change in the presence of the Staple nucleic acid (1-2) (Staple_A) whose guanine repeat sequences were substituted into adenine so as not to form a guanine quadruplex structure (Figure 1(A)).

The results of the fluorescence measurement using the type II oligonucleotides (1-3) and (1-4) are shown in Figure 1(B). That is, a fluorescence signal derived from ThT was obtained in the presence of the G supply-type Staple nucleic acid (1-3) although the fluorescence signal was less intense as compared to that from the type I oligonucleotide, whereas the fluorescence signal did not significantly change in the presence of the Staple nucleic acid (1-4) (Staple_A) whose guanine repeat sequences were substituted into adenine so as not to form a guanine quadruplex structure (Figure 1(B)).

These results show that the oligonucleotide (1-1) and the oligonucleotide (1-3) both functioned as a G supply-type Staple nucleic acid. The fluorescence signal varied depending on the location of the guanine repeat sequence inserted in the G supply-type Staple nucleic acid, and a larger fluorescence signal was obtained by the oligonucleotide designed to insert guanine repeat sequences at around the center region between the first nucleotide sequence and the second nucleotide sequence (Figure 1), thus suggesting that the guanine quadruplex structure formation efficiency depends on the binding mode of the G supply-type Staple nucleic acid.

### (2) Second generation-type Staple nucleic acid binding to 2+1 100nt RNA as target nucleic acid

In this experiment, "2+1 100nt RNA" which is another model target nucleic acid was designed and used. The nucleotide sequence of 2+1 100nt RNA as a target nucleic acid is as follows.

### [Chem. 8]

### <2+1 100nt RNA>

5'_ACACAGGAAACAGCUAUGACCAUGAUUACGCCAAGUUUGCACGCCAGCCGUUCGACGA
UUUAAUACGACUCACUAUAGAUUAGCAUACGCUACUGCAGUGGGUGGGUGUCGACCUAG
AUUAAUGCAAUUCGUGUGAAGAUCCUUUUUGAUAAUCUCAUGACCAAAAUCCCUUAACG
UGAGUUCAUUUCCAGCACCCAAUUGAAGCUUUAAAUGGGUGCAUGCCAUGACUUGCAAG
CUAUUCAGCAGGUAUACAUAGUUGGAAAUCUCUGGAAGAUCCGCGCGUACCGAGUUCUA
AUUCACUGGCCGUCGUUUUACAACGUCGUGACUGGGAAAACCCUGGCGUUACCCAACUU
AAUCGCCUUGCAGCACAUCC_3' (SEQ ID NO: 6)

Boxed region: the guanine repeat sequence used in formation of guanine quadruplex structure
Underlined region: the region where G supply-type Staple nucleic acid is bound

This sequence has three guanine repeat sequences in the target nucleic acid, and can form a guanine quadruplex structure only when one guanine repeat sequences is supplied by the second generation-type Staple nucleic acid of the present invention.

For this target nucleic acid, the following oligonucleotides were designed and created. In the designing of the oligonucleotides, an attempt was made to design oligonucleotides based on a positional relationship between a binding site of a first nucleotide sequence and a binding site of a second nucleotide sequence on the target nucleic acid and guanine repeat sequences on the target nucleic acid. That is, the oligonucleotides of this Example have one guanine repeat sequence located between the first nucleotide sequence and the second nucleotide sequence.

The following oligonucleotides were designed and prepared:
(2-1) G supply-type Staple nucleic acid (for 2+1 100nt RNA): G supply-type Staple nucleic acid which binds to 2+1 100nt RNA as a target nucleic acid, and
(2-2) Staple_A (for 2+1 100nt RNA): an oligonucleotide which was prepared by changing the guanine repeat sequence portions of the (2-1) oligonucleotide to adenine repeat sequences.

### [Chem. 9]

(2-1) G supply-type Staple nucleic acid (for 2+1 100nt RNA) TTGCATTAATCTAGGTCGACTGGGTTTTAAAGCTTCAATTGGGTG (SEQ ID NO: 7)
(2-2) Staple_A (for 2+1 100nt RNA)
   TTGCATTAATCTAGGTCGACTAAATTTTAAAGCTTCAATTGGGTG (SEQ ID NO: 8)
Boxed region: the guanine repeat sequence used in formation of guanine quadruplex structure
Underlined region: the first nucleotide sequence and the second nucleotide sequence

These oligonucleotides were used to perform fluorescence measurement using Thioflavin T (ThT), known to bind to a guanine quadruplex structure and emit fluorescence, for evaluating the function of the G supply-type Staple nucleic acid to form a guanine quadruplex structure on the target nucleic acid.

The fluorescence measurement using Thioflavin T (ThT) was performed by the method described above.

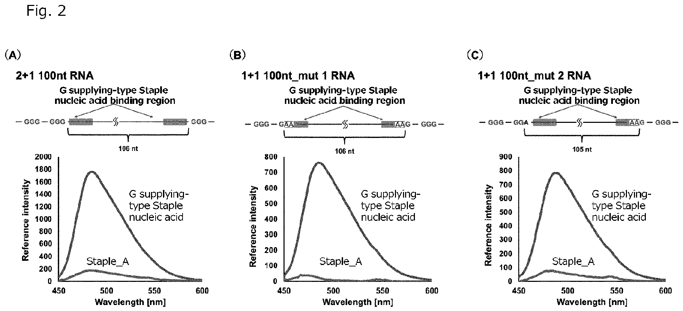

The results of the fluorescence measurement using the oligonucleotides (2-1) and (2-2) are shown in Figure 2(A). That is, a large fluorescence signal derived from ThT was exerted in the presence of the G supply-type Staple nucleic acid (2-1), whereas the fluorescence signal did not significantly change in the presence of the Staple nucleic acid (2-2) (Staple_A) whose guanine repeat sequences were substituted into adenine so as not to form a guanine quadruplex structure (Figure 2(A)).

The results show that the oligonucleotide (2-1) functioned as a G supply-type Staple nucleic acid.

### (3) Second generation-type Staple nucleic acid binding to 1+1 100nt_mut 1 RNA as target nucleic acid

In this experiment, "1+1 100nt_mut 1 RNA" which is another model target nucleic acid was designed and used. The nucleotide sequence of 1+1 100nt_mut 1 RNA as a target nucleic acid is as follows.

### [Chem. 10]

### <1+1 100nt_mut 1 RNA>

5'_ACACAGGAAACAGCUAUGACCAUGAUUACGCCAAGUUUGCACGCCAGCCGUUCGACGA
UUUAAUACGACUCACUAUAGAUUAGCAUACGCUACUGCAGUGGGUGAAUGUCGACCUAG
AUUAAUGCAAUUCGUGUGAAGAUCCUUUUUGAUAAUCUCAUGACCAAAAUCCCUUAACG
UGAGUUCAUUUCCAGCACCCAAUUGAAGCUUUAAGUGGGUGCAUGCCAUGACUUGCAAG
CUAUUCAGCAGGUAUACAUAGUUGGAAAUCUCUGGAAGAUCCGCGCGUACCGAGUUCUA
AUUCACUGGCCGUCGUUUUACAACGUCGUGACUGGGAAAACCCUGGCGUUACCCAACUU
AAUCGCCUUGCAGCACAUCC_3' (SEQ ID NO: 9)

Boxed region: the guanine repeat sequence used in formation of guanine quadruplex structure
Underlined region: the region where G supply-type Staple nucleic acid is bound

This sequence has two guanine repeat sequences in the target nucleic acid, and can form a guanine quadruplex structure only when two guanine repeat sequences are supplied by the second generation-type Staple nucleic acid of the present invention.

For this target nucleic acid, the following oligonucleotides were designed and created. In the designing of the oligonucleotides, an attempt was made to design oligonucleotides based on a positional relationship between a binding site of a first nucleotide sequence and a binding site of a second nucleotide sequence on the target nucleic acid and guanine repeat sequences on the target nucleic acid. That is, the oligonucleotides of this Example have two guanine repeat sequences located between the first nucleotide sequence and the second nucleotide sequence.

The following oligonucleotide was designed and prepared:
(3-1) G supply-type Staple nucleic acid (for 1+1 100nt_mut 1 RNA): G supply-type Staple nucleic acid which binds to 1+1 100nt _mut 1 RNA as a target nucleic acid, and
(3-2) Staple_A (for 1+1 100nt_mut 1 RNA): an oligonucleotide which was prepared by changing guanine repeat sequence portions the (3-1) oligonucleotide to adenine repeat sequences.

### [Chem. 11]

(3-1) G supply-type Staple nucleic acid (for 1+1 100nt_mut 1 RNA) ATTAATCTAGGTCGACATTTTGGGTGGGTTTTAAAGCTTCAATTGGGTG (SEQ ID NO: 10)
(3-2) Staple_A (for 1+1 100nt_mut 1 RNA)
   ATTAATCTAGGTCGACATTTTAAATAAATTTTAAAGCTTCAATTGGGTG (SEQ ID NO: 11)
Boxed region: the guanine repeat sequence used in formation of guanine quadruplex structure
Underlined region: the first nucleotide sequence and the second nucleotide sequence

These oligonucleotides were used to perform fluorescence measurement using Thioflavin T (ThT), known to bind to a guanine quadruplex structure and emit fluorescence, for evaluating the function of the G supply-type Staple nucleic acid to form a guanine quadruplex structure on the target nucleic acid.

The fluorescence measurement using Thioflavin T (ThT) was performed by the method described above.

The results of the fluorescence measurement using the oligonucleotides (3-1) and (3-2) are shown in Figure 2(B). That is, a large fluorescence signal derived from ThT was exerted in the presence of the G supply-type Staple nucleic acid (3-1), whereas the fluorescence signal did not significantly change in the presence of the Staple nucleic acid (3-2) (Staple_A) whose guanine repeat sequences were substituted into adenine so as not to form a guanine quadruplex structure (Figure 2(B)).

The results show that the oligonucleotide (3-1) functioned as a G supply-type Staple nucleic acid.

### (4) Second generation-type Staple nucleic acid binding to 1+1 100nt_mut 2 RNA as target nucleic acid

In this experiment, "1+1 100nt_mut 2 RNA" which is another model target nucleic acid was designed and used. The nucleotide sequence of 1+1 100nt_mut 2 RNA as a target nucleic acid is as follows.

### [Chem. 12]

### <1+1 100nt_mut 2 RNA>

5'_ACACAGGAAACAGCUAUGACCAUGAUUACGCCAAGUUUGCACGCCAGCCGUUCGACGA
UUUAAUACGACUCACUAUAGAUUAGCAUACGCUACUGCAGUGGGUGGAUGUCGACCUAG
AUUAAUGCAAUUCGUGUGAAGAUCCUUUUUGAUAAUCUCAUGACCAAAAUCCCUUAACG
UGAGUUCAUUUCCAGCACCCAAUUGAAGCUUUAAGUGGGUGCAUGCCAUGACUUGCAAG
CUAUUCAGCAGGUAUACAUAGUUGGAAAUCUCUGGAAGAUCCGCGCGUACCGAGUUCUA
AUUCACUGGCCGUCGUUUUACAACGUCGUGACUGGGAAAACCCUGGCGUUACCCAACUU
AAUCGCCUUGCAGCACAUCC_3' (SEQ ID NO: 12)

Boxed region: the guanine repeat sequence used in formation of guanine quadruplex structure
Underlined region: the region where G supply-type Staple nucleic acid is bound

This sequence has two guanine repeat sequences in the target nucleic acid, and can form a guanine quadruplex structure only when two guanine repeat sequences are supplied by the second generation-type Staple nucleic acid of the present invention.

For this target nucleic acid, the following oligonucleotides were designed and created. In the designing of the oligonucleotides, an attempt was made to design oligonucleotides based on a positional relationship between a binding site of a first nucleotide sequence and a binding site of a second nucleotide sequence on the target nucleic acid and guanine repeat sequences on the target nucleic acid. That is, the oligonucleotides of this Example have two guanine repeat sequences located between the first nucleotide sequence and the second nucleotide sequence.

The following oligonucleotides were designed and prepared:
(4-1) G supply-type Staple nucleic acid (for 1+1 100nt_mut 2 RNA): G supply-type Staple nucleic acid which binds to 1+1 100nt_mut 2 RNA as a target nucleic acid, and
(4-2) Staple_A (for 1+1 100nt_mut 2 RNA): an oligonucleotide which was prepared by changing the guanine repeat sequence portions of the (4-1) oligonucleotide to adenine repeat sequences.

### [Chem. 13]

(4-1) G supply-type Staple nucleic acid (for 1+1 100nt mut 2 RNA) GCATTAATCTAGGTCGACTATGTGGTTTAAAGCTTCAATTGGGTGC (SEQ ID NO: 13)
(4-2) Staple_A (for 1+1 100nt_mut 2 RNA)
   GCATTAATCTAGGTCGACTATATAATTTAAAGCTTCAATTGGGTGC (SEQ ID NO: 14)
Boxed region: the guanine repeat sequence used in formation of guanine quadruplex structure
Underlined region: the first nucleotide sequence and the second nucleotide sequence

These oligonucleotides were used to perform fluorescence measurement using Thioflavin T (ThT), known to bind to a guanine quadruplex structure and emit fluorescence, for evaluating the function of the G supply-type Staple nucleic acid to form a guanine quadruplex structure on the target nucleic acid.

The fluorescence measurement using Thioflavin T (ThT) was performed by the method described above.

The results of the fluorescence measurement using the oligonucleotides (4-1) and (4-2) are shown in Figure 2(C). That is, a large fluorescence signal derived from ThT was exerted in the presence of the G supply-type Staple nucleic acid (4-1), whereas the fluorescence signal did not significantly change in the presence of the Staple nucleic acid (4-2) (Staple_A) whose guanine repeat sequences were substituted into adenine so as not to form a guanine quadruplex structure (Figure 2(C)).

The results show that the oligonucleotide (4-1) functioned as a G supply-type Staple nucleic acid.

### (5) Second generation-type Staple nucleic acid binding to 1+1 100nt RNA-2 as target nucleic acid

In this experiment, "1+1 100nt RNA-2" which is another model target nucleic acid was designed and used. The nucleotide sequence of 1+1 100nt RNA-2 as a target nucleic acid is as follows.

### [Chem. 14]

### <1+1 100nt RNA-2>

5'_AUACCGCCUCCUGAAAGUUGUGGCUACAGGACAGGCUUUGGCUGCUUUCUAGAACGU
CGCUCGGAUCCCUUUAAAGGUGGGGAUCUUGACGGAGAGUGCGGGGGAUGAAGGCGGGA
GCUGAGGGCUGGAGAGUCUCUGUUGACAUAGUAACUCUUCAGCUCCGUCUCCCUUGCUC
UCCGCUCUUACGCUUCGCUACCACCAGCGGCCCCGCCUGUGCCCUCUCUGCCCGGGCGCC
CCAGACGCAUCCUCGCGGGGUCUCCUCGGCCUGACCUGCUCAGGUCAAGAUCCUCUUUG
CACCCCCUUAAGUGGUGACUUUUCCCCGGGCCAGUGGGCGAGCCACUUGCGGCGGGCGU
CUGCACCCCCUGCUUCACCGUCGUCCCCUGGGCACCGGUCUGCCCAGGUCCAGUUCGGCC
GCUGACGCGAACCCUCCGCACCGGGUCCCCGCUGGAACUGCCCACUCGGCUCCCCCGGGA
GCGGGGCCCAGGCCAGUCGGGCGUUCCCGCC_3'   (SEQ ID NO: 15)

Boxed region: the guanine repeat sequence used in formation of guanine quadruplex structure
Underlined region: the region where G supply-type Staple nucleic acid is bound

This sequence has two guanine repeat sequences in the target nucleic acid, and can form a guanine quadruplex structure only when two guanine repeat sequences are supplied by the second generation-type Staple nucleic acid of the present invention.

For this target nucleic acid, the following oligonucleotides were designed and created. In the designing of the oligonucleotide, an attempt was made to design oligonucleotides based on a positional relationship between a binding site of a first nucleotide sequence and a binding site of a second nucleotide sequence on the target nucleic acid and guanine repeat sequences on the target nucleic acid. That is, the oligonucleotides of this Example have two guanine repeat sequences located between the first nucleotide sequence and the second nucleotide sequence.

The following oligonucleotides were designed and prepared:
(5-1) G supply-type Staple nucleic acid (for 1+1 100nt RNA-2): G supply-type Staple nucleic acid which binds to 1+1 100nt RNA-2 as a target nucleic acid, and
(5-2) Staple_A (for 1+1 100nt RNA-2): an oligonucleotide which was prepared by changing the guanine repeat sequence portions of the (5-1) oligonucleotide are changed to adenine repeat sequences. This Staple nucleic acid was used in stop assay.

### [Chem. 15]

(5-1) G supply-type Staple nucleic acid (for 1+1 100nt RNA-2) GAGCAGGTCAGGCCGAGGAGTGGGTGGGTCTCCCGGGGGAGCCGAGTGG (SEQ ID NO: 16)
(5-2) Staple_A (for 1+1 100nt RNA-2)
   GAGCAGGTCAGGCCGAGGAGTAAATAAATCTCCCGGGGGAGCCGAGTGG (SEQ ID NO: 17)
Boxed region: the guanine repeat sequence used in formation of guanine quadruplex structure
Underlined region: the first nucleotide sequence and the second nucleotide sequence

### (6) Second generation-type Staple nucleic acid binding to TRPC6 5'UTR RNA as target nucleic acid

In this experiment, a Staple nucleic acid for "TRPC6 5'UTR RNA" which is another target nucleic acid was designed and used. The nucleotide sequence of the Staple nucleic acid for TRPC6 5'UTR RNA as a target nucleic acid is as follows.

### [Chem. 16]

### <TRPC6 5'UTR RNA>

5'_GGGAUACCGCCUCCUGAAAGUUGUGGCUACAGGACAGGCUUUGGCUGCUUUCUAGAA
CGUCGCUCGGAUCCCUUUAAAGGUGGGGAUCUUGACGGAGAGUGCGGGGGAUGAAGGCG
GGAGCUGAGGGCUGGAGAGUCUCUGUUGACAUAGUAACUCUUCAGCUCCGUCUCCCUUG
CUCUCCGCUCUUACGCUUCGCUACCACCAGCGGCCCCGCCUGUGCCCUCUCUGCCCGGGC

GCCCCAGACGCAUCCUCGCGGGGUCUCCUCGGCCUGACCUGCUCAGGUCAAGAUCCUCU
UUGCACCCCCUUAAGUGGUGACUUUUCCCCGGGCCAGUGGGCGAGCCACUUGCGGCGGG
CGUCUGCACCCCCUGCUUCACCGUCGUCCCCUGGGCACCGGUCUGCCCAGGUCCAGUUCG
GCCGCUGACGCGAACCCUCCGCACCGGGUCCCCGCUGGAACUGCCCACUCGGCUCCCCCG
GGAGCGGGGCCCAGGCCAGUCGGGCGUUCCCGCCCCCGGGUACCGAGCUCGAAUUCACUG
GCCGUCGUUUUACAACGUCGUGACUGGGAAAACCCUGGCG_3'   (SEQ ID NO: 18)

Boxed region: the guanine repeat sequence used in formation of guanine quadruplex structure
Underlined region: the region where G supply-type Staple nucleic acid is bound

This sequence has two guanine repeat sequences in the target nucleic acid, and can form a guanine quadruplex structure only when two guanine repeat sequences are supplied by the second generation-type Staple nucleic acid of the present invention.

For this target nucleic acid, the following oligonucleotides were designed and created. In the designing of the oligonucleotide, an attempt was made to design oligonucleotides based on a positional relationship between a binding site of a first nucleotide sequence and a binding site of a second nucleotide sequence on the target nucleic acid and guanine repeat sequences on the target nucleic acid. That is, the oligonucleotides of this Example have two guanine repeat sequences located between the first nucleotide sequence and the second nucleotide sequence.

The following oligonucleotides were designed and synthesized:
(6-1) G supply-type Staple nucleic acid_T (1-1-1) (for TRPC6 5'UTR RNA): G supply-type Staple nucleic acid which binds to TRPC6 5'UTR RNA as a target nucleic acid and in which the arrangement of Ts adjacent to the guanine repeat sequences is one T - G repeat sequence - one T -G repeat sequence - one T,
(6-2) G supply-type Staple nucleic acid_T (2-1-2) (for TRPC6 5'UTR RNA): G supply-type Staple nucleic acid which binds to TRPC6 5'UTR RNA as a target nucleic acid and in which the arrangement of Ts adjacent to the guanine repeat sequences is two Ts - G repeat sequence - one T -G repeat sequence - two Ts,
(6-3) G supply-type Staple nucleic acid_T (3-1-3) (for TRPC6 5'UTR RNA): G supply-type Staple nucleic acid which binds to TRPC6 5'UTR RNA as a target nucleic acid and in which the arrangement of Ts adjacent to the guanine repeat sequences is three Ts - G repeat sequence - one T -G repeat sequence - three Ts,
(6-4) G supply-type Staple nucleic acid_T (1-2-1) (for TRPC6 5'UTR RNA): G supply-type Staple nucleic acid which binds to TRPC6 5'UTR RNA as a target nucleic acid and in which the arrangement of Ts adjacent to the guanine repeat sequences is one T - G repeat sequence - two Ts -G repeat sequence - one T,
(6-5) G supply-type Staple nucleic acid_T (1-3-1): G supply-type Staple nucleic acid which binds to TRPC6 5'UTR RNA as a target nucleic acid and in which the arrangement of Ts adjacent to the guanine repeat sequences is one T - G repeat sequence - three Ts - G repeat sequence - one T,
(6-6) G supply-type Staple nucleic acid_T (2-2-2): G supply-type Staple nucleic acid which binds to TRPC6 5'UTR RNA as a target nucleic acid and in which the arrangement of Ts adjacent to the guanine repeat sequences is two Ts - G repeat sequence - two Ts - G repeat sequence - two Ts,
(6-7) G supply-type Staple nucleic acid_T (2-1-2)_36 mer: an oligonucleotide which was prepared by truncating two bases at the 5' end and two bases at the 3' end of the (6-2) oligonucleotide,
(6-8) G supply-type Staple nucleic acid_T (2-1-2)_32 mer: an oligonucleotide which was prepared by truncating four bases at the 5' end and four bases at the 3' end of the (6-2) oligonucleotide, and
(6-9) G supply-type Staple nucleic acid_T (2-1-2)_28 mer: an oligonucleotide which was prepared by truncating six bases at the 5' end and six bases at the 3' end of the (6-2) oligonucleotide. These Staple nucleic acids were used in stop assay.

### [Chem. 17]

(6-1) G supply-type Staple nucleic acid_T (1-1-1) 
GAGCAGGTCAGGCCGAGGAGTGGGGTGGGGTCTCCCGGGGGAGCCGAGTGG (SEQ ID NO:
19)
(6-2) G supply-type Staple nucleic acid_T (2-1-2) 
GAGCAGGTCAGGCCGAGGAGTTGGGGTGGGGTTCTCCCGGGGGAGCCGAGTGG (SEQ ID
NO: 20)
(6-3) G supply-type Staple nucleic acid_T (3-1-3) 
GAGCAGGTCAGGCCGAGGAGTTTGGGGTGGGGTTTCTCCCGGGGGAGCCGAGTGG (SEQ ID
NO: 21)
(6-4) G supply-type Staple nucleic acid_T (1-2-1) 
GAGCAGGTCAGGCCGAGGAGTGGGGTTGGGGTCTCCCGGGGGAGCCGAGTGG (SEQ ID NO:
22)
(6-5) G supply-type Staple nucleic acid_T (1-3-1) 
[?]
GAGCAGGTCAGGCCGAGGAGTGGGGTTTGGGGTCTCCCGGGGGAGCCGAGTGG (SEQ ID
NO: 23)
(6-6) G supply-type Staple nucleic acid_T (2-2-2) 
GAGCAGGTCAGGCCGAGGAGTTGGGGTTGGGGTTCTCCCGGGGGAGCCGAGTGG (SEQ ID
NO: 24)
(6-7) G supply-type Staple nucleic acid_T (2-1-2)_36 mer GCAGGTCAGGCCGAGGAGTTGGGGTGGGGTTCTCCCGGGGGAGCCGAGT (SEQ ID NO: 25)
(6-8) G supply-type Staple nucleic acid_T (2-1-2)_32 mer AGGTCAGGCCGAGGAGTTGGGGTGGGGTTCTCCCGGGGGAGCCGA (SEQ ID NO: 26)
(6-9) G supply-type Staple nucleic acid_T (2-1-2)_28 mer 
[?]
GTCAGGCCGAGGAGTTGGGGTGGGGTTCTCCCGGGGGAGCC (SEQ ID NO: 27)
(6-10) G supply-type Staple nucleic acid_T (1-0-1) 
GAGCAGGTCAGGCCGAGGAGTGGGG GGGGTCTCCCGGGGGAGCCGAGTGG (SEQ ID NO:
38)
(6-11) G supply-type Staple nucleic acid_T (2-0-2) 
GAGCAGGTCAGGCCGAGGAGTTGGGG GGGGTTCTCCCGGGGGAGCCGAGTGG (SEQ ID
NO: 39)
(6-12) G supply-type Staple nucleic acid_T (3-0-3) 
GAGCAGGTCAGGCCGAGGAGTTTGGGG GGGGTTTCTCCCGGGGGAGCCGAGTGG
(SEQ ID NO: 40)
Boxed region: the guanine repeat sequence used in formation of guanine quadruplex structure
Underlined region: the first nucleotide sequence and the second nucleotide sequence

### (7) Second generation-type Staple nucleic acid binding to TRPC6 5'UTR RNA as target nucleic acid

In this experiment, a Staple nucleic acid for "TRPC6 5'UTR RNA" which is another target nucleic acid was designed and used. The nucleotide sequence of the Staple nucleic acid for TRPC6 5'UTR RNA as a target nucleic acid is as follows.

### [Chem. 18]

### <TRPC6 5'UTR RNA>

5'_GGCUAGCCUCGAGAUACCGCCUCCUGAAAGUUGUGGCUACAGGACAGGCUUUGGCUGC
UUUCUAGAACGUCGCUCGGAUCCCUUUAAAGGUGGGGAUCUUGACGGAGAGUGCGGGGG
AUGAAGGCGGGAGCUGAGGGCUGGAGAGUCUCUGUUGACAUAGUAACUCUUCAGCUCCG
UCUCCCUUGCUCUCCGCUCUUACGCUUCGCUACCACCAGCGGCCCCGCCUGUGCCCUCUC
UGCCCGGGCGCCCCAGACGCAUCCUCGCGGGGUCUCCUCGGCCUGACCUGCUCAGGUCAA
GAUCCUCUUUGCACCCCCUUAAGUGGUGACUUUUCCCCGGGCCAGUGGGCGAGCCACUU
GCGGCGGGCGUCUGCACCCCCUGCUUCACCGUCGUCCCCUGGGCACCGGUCUGCCCAGGU
CCAGUUCGGCCGCUGACGCGAACCCUCCGCACCGGGUCCCCGCUGGAACUGCCCACUCGG

CUCCCCCGGAGCGGGGCCCAGGCCAGUCGGCGUUCCCGCCGAAUUCGCCACC- Firefly
luciferase – IRES – Renilla luciferase _3'    (SEQ ID NO: 28)

Boxed region: the guanine repeat sequence used in formation of guanine quadruplex structure
Underlined region: the region where G supply-type Staple nucleic acid is bound
Dotted line region: the sequence derived from pIRES vector

This sequence has two guanine repeat sequences in the target nucleic acid, and can form a guanine quadruplex structure only when two guanine repeat sequences are supplied by the second generation-type Staple nucleic acid of the present invention.

For this target nucleic acid, the following oligonucleotide was designed and created. In the designing of the oligonucleotide, an attempt was made to design an oligonucleotide based on a positional relationship between a binding site of a first nucleotide sequence and a binding site of a second nucleotide sequence on the target nucleic acid and guanine repeat sequences on the target nucleic acid. That is, the oligonucleotide of this Example has two guanine repeat sequences located between the first nucleotide sequence and the second nucleotide sequence.

The following oligonucleotides were designed and prepared:
(7-1) G supply-type Staple nucleic acid (for TRPC6 5'UTR RNA): G supply-type Staple nucleic acid which binds to TRPC6 5'UTR RNA as a target nucleic acid. The Staple nucleic acid was used in *in vitro* translation assay.

### [Chem. 19]

### (7-1) G supply-type Staple nucleic acid (for TRPC6 5'UTR RNA)

GAGCAGGTCAGGCCGAGGAGTTGGGGTGGGGTTCTCCCGGGGGAGCCGAGTGG (SEQ ID
NO: 29)

Boxed region: the guanine repeat sequence used in formation of guanine quadruplex structure
Underlined region: the first nucleotide sequence and the second nucleotide sequence

### Example 2: Studies on site of formation of guanine quadruplex structure on target nucleic acid by second generation-type Staple nucleic acid

In this Example, stop assay was performed for confirming a site on the target nucleic acid at which a guanine quadruplex structure is formed by the Staple nucleic acid prepared in each of Example 1 (5) and Example 1 (6).

When reverse transcription was performed, full-length complementary DNA (cDNA) is generated from RNA. On the other hand, when reverse transcription is performed on RNA in which a guanine quadruplex structure is present, the guanine quadruplex structure inhibits the cDNA elongation reaction of the reverse transcriptase to generate short cDNA. It is possible to confirm the position of the target nucleic acid in which the guanine quadruplex structure is formed by confirming this cDNA elongation reaction.

In the stop assay, the operations of measuring the location of a guanine quadruplex structure through reverse transcription and identifying the sequence of target DNA by the Sanger method are carried out at the same time, allowing the location on the sequence at which reverse transcription stops to be identified.

In stop assay using "(5-1) G supply-type Staple nucleic acid (for 1+1 100nt RNA-2)" (SEQ ID NO: 16) and "(5-2) Staple_A (for 1+1 100 nt RNA-2)" (SEQ ID NO: 17) for "1+1 100nt RNA-2" (SEQ ID NO: 15), which had been prepared in Example 1, "(5) Second generation-type Staple nucleic acid binding to 1+1 100nt RNA-2 as target nucleic acid", a mixed solution prepared to contain template RNA "1+1 100 nt RNA-2" (SEQ ID NO: 15) (0.3 µM (micro M)), the G supply-type Staple nucleic acid (5-1) (SEQ ID NO: 16) (1.0 µM (micro M)), KCl (100 mM), Tris-HCl buffer (pH 7.6, 50 mM) and 5'-CD4 labeled 3'-DNA primer [5'-CD4-CAC ACA GGA AAC AGC TAT GAC CAT GAT TA-3'] (SEQ ID NO: 30) (0.3 µM (micro M)) at the time of reverse transcription reaction was heated at 90°C for 2 minutes, and cooled to 20°C at 1°C per minute. Thereafter, a reverse transcription reaction solution containing an annealing sample, a Rever Tra Ace reverse transcriptase (TOYOBO, Inc.) (100 units), MgCl₂ (5 mM) and dNTPs (0.86 mM) was reacted at 42°C for 30 minutes, to which 60 units of RNase H was added, and the mixture was reacted at 37°C for 30 minutes. The obtained final reaction solution was analyzed with a capillary sequencer CEQ 8000 (BECKMAN COULTER, Inc.). As a sequence marker, DNA Size Standard-600 (BECKMAN COULTER, Inc.) was used. Stop assay using the same protocol as above was performed as a negative control where the Staple_A nucleic acid (5-2) (SEQ ID NO: 17) was used instead of the G supply-type Staple nucleic acid (5-1) (SEQ ID NO: 16).

Stop assay using "(6-1) G supply-type Staple nucleic acid_T (1-1-1) (for TRPC6 5'UTR RNA)" (SEQ ID NO: 19) for "TRPC6 5'UTR RNA" (SEQ ID NO: 18) which had been prepared in Example 1, "(6) Second generation-type Staple nucleic acid binding to TRPC6 5'UTR RNA as target nucleic acid" was performed in the same manner as in the foregoing stop assay except that template RNA "TRPC6 5'UTR RNA" (SEQ ID NO: 18) (0.3 µM (micro M)), the G supply-type Staple nucleic acid (1.0 µM (micro M)) and 5'-CD4 labeled 3'-DNA primer [5'-CD4-GAG ATT TCC AAC TAT GTA TAC CTG-3'] (SEQ ID NO: 31) (0.3 µM (micro M)) for reverse transcription reaction were used. As a negative control, Stop assay using a Staple_A nucleic acid, obtained by replacing G repeat sequences of the G supply-type Staple nucleic acid by A repeat sequences having the same number of bases, instead of the G supply-type Staple nucleic acid (6-1) (SEQ ID NO: 19), was performed by the same protocol as above.

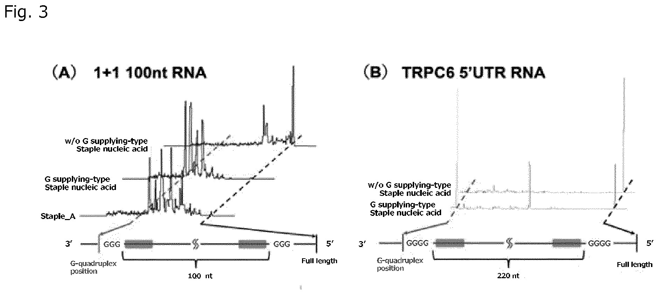

The results of the stop assays are shown in Figure 3. When "1+1 100nt RNA-2" (SEQ ID NO: 15) was a target nucleic acid, the RTase stop signal derived from a guanine quadruplex structure was detected in the presence of the G supply-type Staple nucleic acid (5-1), but was not detected in the Staple_A (5-2) (Figure 3(A)).

When the studies were conducted with the G supply-type Staple nucleic acid (6-1) applied to 5'UTR of TRPC6 gene ("TRPC6 5'UTR RNA" (SEQ ID NO: 18)) as a target nucleic acid, the RTase stop signal was detected in the presence of a G supply-type Staple nucleic acid (Figure 3(B)).

These results show that a guanine quadruplex structure can be formed by the G supply-type Staple nucleic acid not only in the model sequence (1+1 100nt RNA) (Figure 3(A)), but also in the gene (TRPC6) (Figure 3(B)).

In this Example, for further examining (A) how the function of the Staple nucleic acid is affected by the number of T linkers in the G supply-type Staple nucleic acid and (B) how the function of the Staple nucleic acid is affected when the length of the G supply-type Staple nucleic acid itself is changed, stop assay was performed in the same manner as described above, using "TRPC6 5'UTR RNA" (SEQ ID NO: 18) in Example 1 (6) as a target nucleic acid, and using the G supply-type Staple nucleic acids (6-1) to (6-6) and (6-10) to (6-12) (SEQ ID NOS: 19 to 24 and 38 to 40, respectively) for (A), and the G supply-type Staple nucleic acid (6-2) (SEQ ID NO: 20) or the G supply-type Staple nucleic acids (6-7) to (6-9) (SEQ ID NOS: 25 to 27, respectively) obtained by truncating the length of the first nucleotide sequence and the second nucleotide sequence of the G supply-type Staple nucleic acid (6-2) for (B).

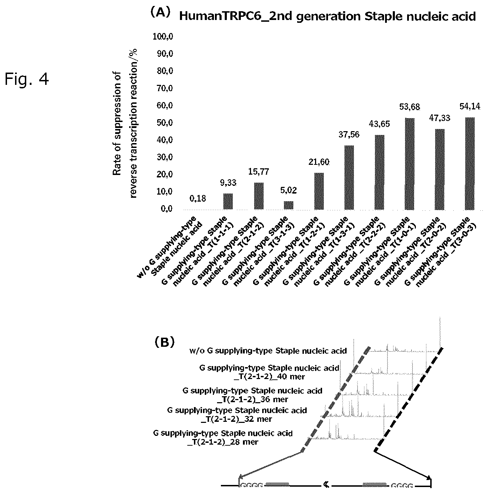

Changing the length of the T linker between the target nucleic acid recognition sequence and the guanine repeat sequence in the G supply-type Staple nucleic acid revealed that the reverse transcription reaction of RTase was inhibited regardless of the structures in which the number of T linkers is 0 to 3 (Figure 4(A)). In addition, when the length of the first nucleotide sequence and the second nucleotide sequence which are target nucleic acid recognition regions was truncated, a RTase stop signal was not detected when the sequence was shorter than 32 bases in length (Figure 4(B)).

These results suggest that the reverse transcription inhibitory effect on RTase does not depend on the length of the linker(s) between the G repeat sequences of the G supply-type Staple nucleic acid, but depends on the stability (length) of a duplex formed between the G supply-type Staple nucleic acid and the target nucleic acid.

### Example 3: Studies of downregulating action of second generation-type Staple nucleic acid on protein expression

In this Example, whether a downregulating action on protein expression was actually generated using the second generation-type Staple nucleic acid prepared in Example 1 was confirmed.

The "(7-1) G supply-type Staple nucleic acid (for TRPC6 5'UTR RNA)" (SEQ ID NO: 29) for "TRPC6 5'UTR RNA" (SEQ ID NO: 28), which had been prepared in Example 1, "(7) Second generation-type Staple nucleic acid recognizing TRPC6 5'UTR RNA as a target nucleic acid", was used, as a target nucleic acid and a G supply-type Staple nucleic acid for the target nucleic acid, to evaluate the effect of a guanine quadruplex structure formed by the G supply-type Staple nucleic acid for each target nucleic acid in (7) TRPC6 5'UTR RNA on the protein translation reaction by *in vitro* translation assay.

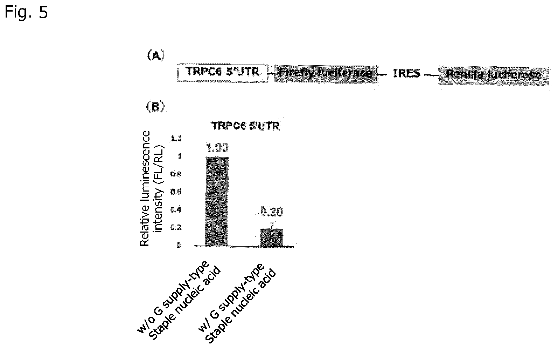

The effect of the G supply-type Staple nucleic acid on the protein translation reaction from each target nucleic acid were examined using two types of reporter genes (connected to the target nucleic acid) which are Firefly luciferase (FL) translated in a Cap-dependent manner and Renilla luciferase (RL) translated via IRES (the outline is shown in Figure 5(A)). By comparing emission signals derived from FL and RL, the effect of the constructed guanine quadruplex structure on gene expression can be quantitatively evaluated.

The evaluation through *in vitro* translation was conducted using each target nucleic acid as a mRNA template (1 µg (microgram)), with which 20 µL (microliter) of a cell-free protein expression mixed solution (RTS 100 Wheat Germ CECF Kit, 5 Prime, Inc) with or without 0.14 µM (micro M) of the G supply-type Staple nucleic acid was reacted at 24°C for 150 minutes. The luciferase activity was examined using Luciferase Assay System (Promega, Inc.), Renilla Luciferase Assay System (Promega, Inc.) and POWERSCAN·H1 microplate reader (BioTek, Inc.).

As a result of *in vitro* translation, the translation efficiency of a transcript having the sequence of each gene decreased in the presence of the G supply-type Staple nucleic acid (Figure 5(B)). These results show that the expression of a target gene can be suppressed using the G supply-type Staple nucleic acid in vitro.

### Example 4: Studies of Staple nucleic acid of invention on target nucleic acid stabilizing action

In this Example, whether a target nucleic acid stabilizing action was exerted using the first generation-type Staple nucleic acid and the second generation-type Staple nucleic acid was confirmed.

The research group of the present inventors had succeeded in development of a compound RGB-1 which selectively binds to a guanine quadruplex structure stabilize it (JACS, 2016). It was found that addition of RGB-1 to cells upregulated the protein expression level in some genes, and that a search of the guanine quadruplex structure by Stop Assay which enables identification of a location at which a guanine quadruplex structure is formed revealed that RGB-1 induced the formation of the guanine quadruplex structure at 3'UTR of specific mRNA.

Thus, in this Example, on the assumption that when a guanine quadruplex structure is present in a target nucleic acid, exonuclease may be physically blocked to acquire resistance, a Staple nucleic acid targeting the 3'UTR region of the target nucleic acid was designed and created to improve the stability of the target nucleic acid (hence, to enhance the expression of protein).

In this Example, a target nucleic acid having the following sequence (SEQ ID NO: 32) was designed and used. In this Example, further a Staple nucleic acid to be applied to the target nucleic acid was created as follows.

### [Chem. 20]

### <PTC opti GFP>

GATTAGCATACGCTACTGCAGTGGGTGGGTGTCGACCTAGATTAATGCAATTCGTGT
GAAGATCCTTTTTGATAATCTCATGACCAAAATCCCTTAACGTGAGTTCATTTCCAGCA
CCCAATTGAAGCTTTGGGTGGGTGCATGCCATGACTTGCAAGCTATTCAGCAGGTAT
ACATAGTTGGAA (SEQ ID NO: 32)

Boxed region: the guanine repeat sequence used in formation of guanine quadruplex structure
Underlined region: the region where Staple nucleic acid is bound
Staple nucleic acid A1 (first generation-type Staple nucleic acid)
   TTGTGGCCGTTTACGT-TCGCCGGACACGCT (SEQ ID NO: 33)
Boxed region: the guanine repeat sequence used in formation of guanine quadruplex structure
Underlined region: the first nucleotide sequence and the second nucleotide sequence

The created template RNA was used as a target nucleic acid. The template RNA and the Staple nucleic acid were incubated at 90°C for 2 minutes with KCl (150 mM) and Tris-HCl buffer (pH 7.6) (20 mM), and allowed to cool to 20°C at 1°C/min, thereby being annealed (template RNA : Staple = 1 : 1.25). The annealing solution was used to prepare a measurement solution (total volume: 150 µl (microliter)) such that it contained RNA (0.04 µM (micro M)), 1 × RNaseR Buffer, and RNase R (0.01 units) or milliQ (control). An absorption spectrum at 260 nm was measured at 37°C for 1 hour using UV/VIS Spectrophotometer V-560 (JASCO). This was repeated three times to obtain N = 3. In this procedure, decomposition of the target nucleic acid can be detected as the UV absorption value increases.

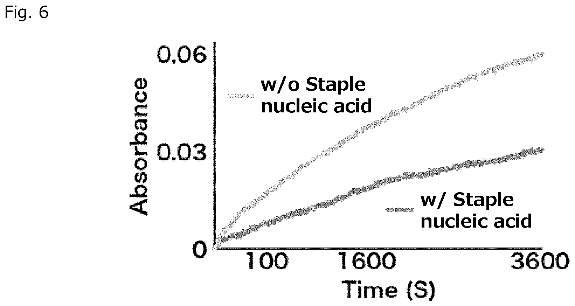

It could be confirmed that, as a result of inducing formation of a guanine quadruplex structure in 3'UTR of the target nucleic acid by introduction of the Staple nucleic acid, the stability of the target nucleic acid was markedly improved even in the presence of RNaseR (Figure 6). Therefore, the stability was markedly improved due to the presence of the Staple nucleic acid. This resulted in an upregulation in protein expression level.

### Example 5: Studies of Staple nucleic acid of invention on ribosomal shunting action in single target nucleic acid

In this Example, it was confirmed that the first generation-type Staple nucleic acid was used to exert an action that causes ribosomal shunting over sub-sequences of a single target nucleic acid.

It is known that ribosomes, when their progress of a reaction is blocked by a highly thermostable, bulky higher-order structure, takes a strategy, called ribosomal shunting, of skipping over (bypassing) the structure and continuing protein translation. In this Example, a technique was established for controlling ribosomal shunting, and bypassing the stop codon in a single target nucleic acid.

In this Example, a target nucleic acid having the following sequences were designed (SEQ ID NO: 34 for PTC GFP and SEQ ID NO: 35 for Δ (delta) typel GFP) and used. The mRNA of PTC GFP is characterized in that "TGA" present between the first nucleotide sequence and the second nucleotide sequence, represented as the underlined letters, functions as a stop codon, and translation stops at the codon.

### [Chem. 21]

### <PTC GFP>

ATGGTGAGCAAGGGCGAGGAGCTGTTCACCGGGGTGGTGCCCATCCTGGTCGTGGGGGACG
GGGACGTAAACGGCCACAAGTGAAGCGTGTCCGGCGAGGGGGAGGGGGATGCCACCTACG
GCAAGCTGACCCTGAAGTTCATCTGCACCACCGGCAAGCTGCCCGTGCCCTGGCCCACCCTC
GTGACCACCCTGACCTACGGCGTGCAGTGCTTCAGCCGCTACCCCGACCACATGAAGCAGCA
CGACTTCTTCAAGTCCGCCATGCCCGAAGGCTACGTCCAGGAGCGCACCATCTTCTTCAAGG
ACGACGGCAACTACAAGACCCGCGCCGAGGTGAAGTTCGAGGGCGACACCCTGGTGAACCG
CATCGAGCTGAAGGGCATCGACTTCAAGGAGGACGGCAACATCCTGGGGCACAAGCTGGAGT
ACAACTACAACAGCCACAACGTCTATATCATGGCCGACAAGCAGAAGAACGGCATCAAGGTG
AACTTCAAGATCCGCCACAACATCGAGGACGGCAGCGTGCAGCTCGCCGACCACTACCAGCA
GAACACCCCCATCGGCGACGGCCCCGTGCTGCTGCCCGACAACCACTACCTGAGCACCCAGT
CCGCCCTGAGCAAAGACCCCAACGAGAAGCGCGATCACATGGTCCTGCTGGAGTTCGTGACC
GCCGCCGGGATCACTCTCGGCATGGACGAGCTGTACAAGTAA (SEQ ID NO: 34)

### <Δ (delta) type1 GFP>

ATGGTGAGCAAGGGCGAGGAGCTGTTCACCGGGGTGGTGCCCATCCTGGTCGCCACCTACG
GCAAGCTGACCCTGAAGTTCATCTGCACCACCGGCAAGCTGCCCGTGCCCTGGCCCACCCTC
GTGACCACCCTGACCTACGGCGTGCAGTGCTTCAGCCGCTACCCCGACCACATGAAGCAGCA
CGACTTCTTCAAGTCCGCCATGCCCGAAGGCTACGTCCAGGAGCGCACCATCTTCTTCAAGG
ACGACGGCAACTACAAGACCCGCGCCGAGGTGAAGTTCGAGGGCGACACCCTGGTGAACCG
CATCGAGCTGAAGGGCATCGACTTCAAGGAGGACGGCAACATCCTGGGGCACAAGCTGGAGT
ACAACTACAACAGCCACAACGTCTATATCATGGCCGACAAGCAGAAGAACGGCATCAAGGTG
AACTTCAAGATCCGCCACAACATCGAGGACGGCAGCGTGCAGCTCGCCGACCACTACCAGCA
GAACACCCCCATCGGCGACGGCCCCGTGCTGCTGCCCGACAACCACTACCTGAGCACCCAGT
CCGCCCTGAGCAAAGACCCCAACGAGAAGCGCGATCACATGGTCCTGCTGGAGTTCGTGACC
GCCGCCGGGATCACTCTCGGCATGGACGAGCTGTACAAGTAA (SEQ ID NO: 35)

Boxed region: the guanine repeat sequence used in formation of guanine quadruplex structure
Underlined region: the region where Staple nucleic acid is bound

In this Example, Staple nucleic acids that are applied to the target nucleic acid were created as follows. Here, the Staple nucleic acids are of a first generation-type Staple nucleic acid, and both the Staple nucleic acid B1 and the Staple nucleic acid B2 can bind to the target nucleic acid of PTC GFP, but do not bind to Δ (delta) type1 GFP. Both the Staple nucleic acids were designed to cause ribosomal shunting so as to skip over the stop codon. The Staple nucleic acid B1 is truncated by three bases on the 3' end side (GCT) as compared to the Staple nucleic acid B2.

### [Chem. 22]

Staple nucleic acid B1
   TTGTGGCCGTTTACGT TCGCCGGACAC (SEQ ID NO: 36)
Staple nucleic acid B2
   TTGTGGCCGTTTACGT TCGCCGGACACGCT (SEQ ID NO: 37)
Boxed region: the guanine repeat sequence used in formation of guanine quadruplex structure
Underlined region: the first nucleotide sequence and the second nucleotide sequence

mRNA obtained by transcription of the sequence PTC GFP (SEQ ID NO: 34) or Δ (delta) type1 GFP (SEQ ID NO: 35) was used as a template (1 µg (microgram)), with which 20 µL (microliter) of a cell-free protein expression mixed solution (RTS 100 Wheat Germ CECF Kit, 5 Prime, Inc) with or without 0.14 µM (micro M) of each Staple nucleic acid was reacted at 24°C for 150 minutes. The obtained RNA was used as a mRNA template (1 µg (microgram)), with which 20 µL (microliter) of a cell-free protein expression mixed solution (RTS 100 Wheat Germ CECF Kit, 5 Prime, Inc) with or without 0.14 µM (micro M) of the Staple nucleic acid was reacted at 24°C for 150 minutes. Fluorescence measurement was performed using a fluorescence spectrophotometer (Jasco FP-8500) under the condition of 25°C with the excitation wavelength set to 480 nm.

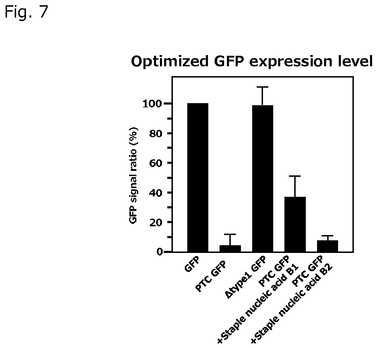

The results are shown in Figure 7. In this figure, the GFP signal intensities of PTC GFP (without Staple nucleic acid), Δ (delta) type1 GFP (without Staple nucleic acid), PTC GFP (with Staple nucleic acid B1) and PTC GFP (with Staple nucleic acid B2) are compared where the GFP signal intensity when mRNA of GFP is expressed is defined as 100. The results show that the signal intensity of PTC GFP (without Staple nucleic acid) was 3% of that of GFP, whereas the signal intensities of PTC GFP (with Staple nucleic acid B1) and PTC GFP (with Staple nucleic acid B2) increased to about 37% and about 8%, respectively.

### Example 6: Studies of first generation-type Staple nucleic acid of invention on target nucleic acid stabilizing action

In this Example, whether when the first generation-type Staple nucleic acid was applied to mRNA as a target nucleic acid, a stabilizing action on the target nucleic acid was exerted, and as a result, expression of protein was enhanced was confirmed.

### (6-1) mRNA stabilizing action

As described in Example 4 above, the first generation-type Staple nucleic acid of the present invention was shown to be capable of exhibiting a target nucleic acid stabilizing action in a cell-free system.

In this Example, a target nucleic acid having the following sequence "Random sequence_2+2 100nt" (a target nucleic acid having a sequence obtained by linking Random sequence and 2+2 100nt) was designed (SEQ ID NO: 41) and used. As a control, "Random sequence_2+2 100nt MutA" obtained by substituting four guanine repeat sequences of the target nucleic acid "Random sequence_2+2 100nt" to "AAA" was designed (SEQ ID NO: 42).

### [Chem. 23]

### <Random sequence_2+2 100nt>

GGGACAGCUCGAAUUAAGAAUAUGUUUCCUUGUUUAUCAGAUACAUCACUGCUUG
AUGAAGCAAGGAAGAUAUACAUGAAAAUUUUAAAAAUACAUAUCGCUGACUUCAU
GGAAUGGACAUCCUGUAUAAGCACUGAAAAACAACAACACAAUAACACUAAAAUUU
UAGGCACUCUUAAAUGAUCUGCCUCUAAAAGCGUUGGAUGUAGCAUUAUGCAAUU
AGGUUUUUCCUUAUUUGCUUCAUUGUACUACCUGUGUAUAUAGUUUUUACCUUU
UAUGUAGCACAUAAACUUUAAAAAAAAAAAAACAAAAUAAAACUGAGGAACUGACG
UGGAGCGGGGUAUGAAGAGCUUGCUUUGAUUUACAGCAAGUAGAUAAAUAUUUGA
CUUGCAUGAAGAGAAGCAAUUUUGGGGAAGGGUUUGAAUUGUUUUCUUUAAAGA
UGUAAUGUCCCUUUCAGAGACAGCUGAUACUUCAUUUAAAAAAAUCACAAAAAUU
UGAACACUGGCUAAAGAUAAUUGCUAUUUAUUUUUACAAGAAGUUUAUUCUCAU
UUGGGAGAUCUGGUGAUCUCCCAAGCUAUCUAAAGUUUGUUAGAUAGCUGCAUG
UGGCUUUUUUAAAAAAGCAACAGAAACCUAUCCUCACUGCCCUCCCCAGUCUCUC
UUAAAGUUGGAUUAGCAUACGCUACUGCAGUGGGUGGGUGUCGACCUAGAUUAA
UGCAAUUCGUGUGAAGAUCCUUUUUGAUAAUCUCAUGACCAAAAUCCCUUAACGU
GAGUUCAUUUCCAGCACCCAAUUGAAGCUUUGGGUGGGUGCAUGCCAUGACUUG
CAAGCUAUUCAGCUCUAGAGUCGACCUGCAGG (SEQ ID NO: 41)

Grayly shaded region: 2+2 sequence
Boxed region: the guanine repeat sequence used in formation of guanine quadruplex structure
Underlined region: the region where Staple nucleic acid is bound

### <Random sequence_2+2 100nt MutA>

GGGACAGCUCGAAUUAAGAAUAUGUUUCCUUGUUUAUCAGAUACAUCACUGCUUG
AUGAAGCAAGGAAGAUAUACAUGAAAAUUUUAAAAAUACAUAUCGCUGACUUCAU
GGAAUGGACAUCCUGUAUAAGCACUGAAAAACAACAACACAAUAACACUAAAAUUU
UAGGCACUCUUAAAUGAUCUGCCUCUAAAAGCGUUGGAUGUAGCAUUAUGCAAUU
AGGUUUUUCCUUAUUUGCUUCAUUGUACUACCUGUGUAUAUAGUUUUUACCUUU
UAUGUAGCACAUAAACUUUAAAAAAAAAAAAACAAAAUAAAACUGAGGAACUGACG
UGGAGCGGGGUAUGAAGAGCUUGCUUUGAUUUACAGCAAGUAGAUAAAUAUUUGA
CUUGCAUGAAGAGAAGCAAUUUUGGGGAAGGGUUUGAAUUGUUUUCUUUAAAGA
UGUAAUGUCCCUUUCAGAGACAGCUGAUACUUCAUUUAAAAAAAUCACAAAAAUU
UGAACACUGGCUAAAGAUAAUUGCUAUUUAUUUUUACAAGAAGUUUAUUCUCAU
UUGGGAGAUCUGGUGAUCUCCCAAGCUAUCUAAAGUUUGUUAGAUAGCUGCAUG
UGGCUUUUUUAAAAAAGCAACAGAAACCUAUCCUCACUGCCCUCCCCAGUCUCUC
UUAAAGUUGGAUUAGCAUACGCUACUGCAGUAAAUAAAUGUCGACCUAGAUUAAU
GCAAUUCGUGUGAAGAUCCUUUUUGAUAAUCUCAUGACCAAAAUCCCUUAACGUG
AGUUCAUUUCCAGCACCCAAUUGAAGCUUUAAAUAAAUGCAUGCCAUGACUUGCA
AGCUAUUCAGCUCUAGAGUCGACCUGCAGG (SEQ ID NO: 42)

Grayly shaded region: 2+2 sequence
Boxed region: the region obtained by changing guanine repeat sequence to AAA Underlined region: the region where Staple nucleic acid is bound

Further in this Example, a first generation-type Staple nucleic acid to be applied to the target nucleic acid was created as follows. For this configuration, the Staple nucleic acid is designed to form a guanine quadruplex structure in a region corresponding to 3'UTR of the target nucleic acid.

### [Chem. 24]

### Staple nucleic acid A2 (first generation-type Staple nucleic acid)

TTGCATTAATCTAGGTCGAC AAGCTTCAATTGGGTGCTGG (SEQ ID NO: 43)
Boxed region: the guanine repeat sequence used in formation of guanine quadruplex structure
Underlined region: the first nucleotide sequence and the second nucleotide sequence

The created template RNA was used as a target nucleic acid. The template RNA and the Staple nucleic acid were incubated at 90°C for 2 minutes with KCl (150 mM) and Tris-HCl buffer (pH 7.6) (10 mM), and allowed to cool to 20°C at 1°C/min, thereby being annealed (template RNA : Staple = 1 : 1.25). The annealing solution was used to prepare a measurement solution (total volume: 150 µl (microliter)) such that it contained RNA (0.04 µM (micro M)), 1 × RNaseR Buffer, RNase R (0.1 units) or milliQ (control). An absorption spectrum at 260 nm was measured at 37°C for 1 hour using UV/VIS Spectrophotometer V-560 (JASCO). This was repeated three times to obtain N = 3. In this procedure, decomposition of the target nucleic acid can be detected as the UV absorption value increases.

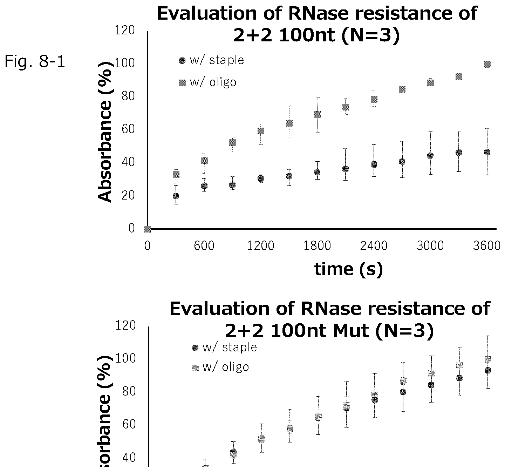

It could be confirmed that, as a result of inducing formation of a guanine quadruplex structure on the 3' end side of the target nucleic acid "Random sequence_2+2 100nt" by introduction of the Staple nucleic acid, the resistance of the target nucleic acid to 3'->5' exonuclease activity was improved, and the stability of the target nucleic acid was markedly improved even in the presence of RNaseR (Figure 8-1, upper chart). On the other hand, it was shown that, in "Random sequence_2+2 100nt MutA" which does not contain a guanine repeat sequence, a guanine quadruplex structure was not formed, and therefore in the presence of RNaseR, "Random sequence_2+2 100nt MutA" was decomposed by 3'→5' exonuclease activity to the same degree as in the case of a negative control ("w/ oligo" group) (Figure 8-1, lower chart). Therefore, the stability was markedly improved due to the presence of the Staple nucleic acid.

### (6-2) Protein expression level upregulating action

In this Example, the ability of the Staple nucleic acid of the present invention to form a guanine quadruplex structure in a target nucleic acid even in cells, and whether, as a result, the stability of the target nucleic acid is improved (hence, the protein expression is upregulated) even in the cells were confirmed.

In this Example, a target nucleic acid having the following sequence "Firefly_2+2" (firefly luciferase gene sequence to which 2+2 sequence is connected) was designed (SEQ ID NO: 44) and used.

### [Chem. 25]

### <Firefly_2+2>

ATGGCCGATGCTAAGAACATTAAGAAGGGCCCTGCTCCCTTCTACCCTCTGGAGGATGGCAC
CGCTGGCGAGCAGCTGCACAAGGCCATGAAGAGGTATGCCCTGGTGCCTGGCACCATTGCCT
TCACCGATGCCCACATTGAGGTGGACATCACCTATGCCGAGTACTTCGAGATGTCTGTGCGC
CTGGCCGAGGCCATGAAGAGGTACGGCCTGAACACCAACCACCGCATCGTGGTGTGCTCTGA
GAACTCTCTGCAGTTCTTCATGCCAGTGCTGGGCGCCCTGTTCATCGGAGTGGCCGTGGCCC
CTGCTAACGACATTTACAACGAGCGCGAGCTGCTGAACAGCATGGGCATTTCTCAGCCTACC
GTGGTGTTCGTGTCTAAGAAGGGCCTGCAGAAGATCCTGAACGTGCAGAAGAAGCTGCCTAT
CATCCAGAAGATCATCATCATGGACTCTAAGACCGACTACCAGGGCTTCCAGAGCATGTACA
CATTCGTGACATCTCATCTGCCTCCTGGCTTCAACGAGTACGACTTCGTGCCAGAGTCTTTCG
ACAGGGACAAAACCATTGCCCTGATCATGAACAGCTCTGGGTCTACCGGCCTGCCTAAGGGC
GTGGCCCTGCCTCATCGCACCGCCTGTGTGCGCTTCTCTCACGCCCGCGACCCTATTTTCGGC
AACCAGATCATCCCCGACACCGCTATTCTGAGCGTGGTGCCATTCCACCACGGCTTCGGCAT
GTTCACCACCCTGGGCTACCTGATTTGCGGCTTTCGGGTGGTGCTGATGTACCGCTTCGAGG
AGGAGCTGTTCCTGCGCAGCCTGCAAGACTACAAAATTCAGTCTGCCCTGCTGGTGCCAACC
CTGTTCAGCTTCTTCGCTAAGAGCACCCTGATCGACAAGTACGACCTGTCTAACCTGCACGAG
ATTGCCTCTGGCGGCGCCCCACTGTCTAAGGAGGTGGGCGAAGCCGTGGCCAAGCGCTTTCA
TCTGCCAGGCATCCGCCAGGGCTACGGCCTGACCGAGACAACCAGCGCCATTCTGATTACCC
CAGAGGGCGACGACAAGCCTGGCGCCGTGGGCAAGGTGGTGCCATTCTTCGAGGCCAAGGT
GGTGGACCTGGACACCGGCAAGACCCTGGGAGTGAACCAGCGCGGCGAGCTGTGTGTGCGC
GGCCCTATGATTATGTCCGGCTACGTGAATAACCCTGAGGCCACAAACGCCCTGATCGACAA
GGACGGCTGGCTGCACTCTGGCGACATTGCCTACTGGGACGAGGACGAGCACTTCTTCATCG
TGGACCGCCTGAAGTCTCTGATCAAGTACAAGGGCTACCAGGTGGCCCCAGCCGAGCTGGAG
TCTATCCTGCTGCAGCACCCTAACATTTTCGACGCCGGAGTGGCCGGCCTGCCCGACGACGA
TGCCGGCGAGCTGCCTGCCGCCGTCGTCGTGCTGGAACACGGCAAGACCATGACCGAGAAG
GAGATCGTGGACTATGTGGCCAGCCAGGTGACAACCGCCAAGAAGCTGCGCGGCGGAGTGG
TGTTCGTGGACGAGGTGCCCAAGGGCCTGACCGGCAAGCTGGACGCCCGCAAGATCCGCGA
GATCCTGATCAAGGCTAAGAAAGGCGGCAAGATCGCCGTGTAAGATTAGCATACGCTACTGC
AGTGGGTGGGTGTCGACCTAGATTAATGCAATTCGTGTGAAGATCCTTTTTGATAATCTCATG
ACCAAAATCCCTTAACGTGAGTTCATTTCCAGCACCCAATTGAAGCTTTGGGTGGGTGCATG
CCATGACTTGCAAGCTATTCAG (SEQ ID NO: 44)

Grayly shaded region: the firefly luciferase sequence
Grayly shaded and boxed region: the start codon/stop codon of firefly luciferase
Non-gray region: 2+2 sequence
Boxed region: the guanine repeat sequence used in formation of guanine quadruplex structure
Underlined region: the region where Staple nucleic acid is bound

Fhrther in this Example, a first generation-type Staple nucleic acid to be applied to the target nucleic acid was created as follows. The Staple nucleic acid with this configuration is designed such that, by the Staple nucleic acid, a guanine quadruplex structure is formed in a region corresponding to 3'UTR of the target nucleic acid.

### [Chem. 26]

### Staple nucleic acid A3 (first generation-type Staple nucleic acid)

TTGCATTAATCTAGGTCGAC AAGCTTCAATTGGGTGCTGG (SEQ ID NO: 45)
Boxed region: the guanine repeat sequence used in formation of guanine quadruplex structure
Underlined region: the first nucleotide sequence and the second nucleotide sequence

HEK 293T cell #16 cells were seeded on a 96-well plate at a cell density of 2 × 10⁴ cells/ml, and cultured for 72 hours in 100 µl (microliter) of D-MEM medium containing 10% FBS and 1% penicillin. When the confluency was confirmed to be 80%, the medium was changed to 100 µl (microliter) of D-MEM medium containing 10% FBS, followed by culture for 3 hours.

The cells were divided into two groups, a group to which the Staple nucleic acid was to be applied and a group to which the Staple nucleic acid was not to be applied. In accordance with the protocol of FuGENE (registered trademark) HD Transfection Reagent (Promega E2311), 1 µg (microgram) of pSuper which expresses the sequence of the Staple nucleic acid under control of the H1 promotor was added to the medium for the former group, and 1 µg (microgram) of a vector solution of blank pSuper free of inserts was added to the medium for the latter group. The cells were cultured in a 5% CO₂ atmosphere at 37°C for 24 hours.

Thereafter, for both the groups, 1 µg (microgram) of a solution of pBI-CMV1 MCS1 which expresses "Firefly_2+2" under control of the CMV promotor was added to the medium in accordance with the protocol of FuGENE (registered trademark) HD Transfection Reagent (Promega E2311), and the cells were cultured in a 5% CO₂ atmosphere at 37°C while being sampled after 8 hours, 12 hours and 24 hours.

For the protein expression of firefly luciferase, the luciferase emission intensity when "Firefly_2+2" was expressed for 24 hours in the group to which the Staple nucleic acid had not been added was defined as 100%, and relative to this value, the luciferase emission intensity of each sample was measured, from which a protein expression level ratio (%) was calculated.

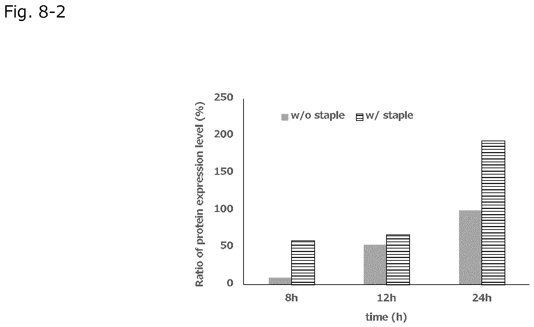

The results are shown in Figure 8-2. This figure also demonstrates that, due to the presence of the Staple nucleic acid, the guanine quadruplex structure formed in the UTR region at the 3' end upregulates the protein expression level in cells.

### Example 7: Studies of second generation-type Staple nucleic acid of invention on target nucleic acid stabilizing action

In this Example, whether the second generation-type Staple nucleic acid was used to exert a target nucleic acid stabilizing action even when cells were used was confirmed.

### (7-1) mRNA stabilizing action

As described above, the second generation-type Staple nucleic acid of the present invention was shown to be capable of exhibiting a target nucleic acid stabilizing action in a cell-free system.

In this Example, for a Neurofibromin 1 (NF1), a target nucleic acid having the following sequence "a part of NF1 3'UTR" (a target nucleic acid having the gene sequence of NF1) was designed (SEQ ID NO: 46) and used.

### [Chem. 27]

### <Part of NF1 3'UTR>

CACAGACAAGGTACTTGGGGGGGAGGGCAGGGAAATTTCATATTTTATAGTGGATTCTTAAG
AAATACTAACACTTGAGTATTAGCAATAATTACAGGAAAATAAGTGCGACCACATATATCTTA
ACATTACTGAATTAAAACTATGGCTTCTAAGTCCTTATCCAAACTCAGTCATCCAAACTAGTTT
ATTTTTTTCTCCAGTTGATTATCTTTTAATTTTTAATTTTGCTAAAGGTGGTTTTTTTGTGTTTT
GTTTTTTGTAAACCAAAACTATACTAAGTATAGTAATTATATATATATATATATTTTTTCCCCT
CCCCCTCTTCTTTCCTAACTAATTCTGAGCAGGGTAATCAGTGAACAAAGTGTTGAAAATTGT
TCCCAGAAGGTAATTTTCATAGATGTTTGCATTAGCTCCATAGCAAAATGGAATGGTACGTGA
CATTTAGGGTAGCTGATATTTTTATTTTGTT (SEQ ID NO: 46)

Boxed region: the guanine repeat sequence used in formation of guanine quadruplex structure
Gray underlined region: the region where first generation-type Staple nucleic acid is bound
Underlined region: the region where second generation-type Staple nucleic acid is bound

Further in this Example, a second generation-type Staple nucleic acid to be applied to the target nucleic acid was created as follows. The Staple nucleic acid with this configuration is designed such that two guanine repeat sequences are supplied by the Staple nucleic acid, which are used with two guanine repeat sequences present in 3'UTR of the target nucleic acid to form a guanine quadruplex structure in a region corresponding to 3'UTR of the target nucleic acid. In cells, the sequence of the Staple nucleic acid is generally transcribed by RNA pol III using a U6 promotor, but at this time, since the consecutive sequence of T causes RNA polymerase to terminate the transcription, a part of T was replaced by A for use in cells.

### [Chem. 28]

### Staple nucleic acid A4 (first generation-type Staple nucleic acid)

CCACTATAAAATATGAAATT GCTCAGAATTAGTTAGGAAA (SEQ ID NO: 47)
Boxed region: the guanine repeat sequence used in formation of guanine quadruplex structure
Underlined region: the first nucleotide sequence and the second nucleotide sequence

### Staple nucleic acid A5 (second generation-type Staple nucleic acid

CAACACTTTGTTCACTGATTTTGGGTGGGTTAAATGTCACGTACCATTCCA (SEQ ID NO: 48)

Boxed region: the guanine repeat sequence used in formation of guanine quadruplex structure
Underlined region: the first nucleotide sequence and the second nucleotide sequence

The stabilization of mRNA was examined basically in accordance with the method described in Example 6 (6-1). The created template RNA was used as a target nucleic acid. The template RNA and the Staple nucleic acid were incubated at 90°C for 2 minutes with KCl (150 mM) and Tris-HCl buffer (pH 7.6) (10 mM), and allowed to cool to 20°C at 1°C/min, thereby being annealed (template RNA : Staple = 1 : 1.25). The annealing solution was used to prepare a measurement solution (total volume: 150 µl (microliter)) such that it contained RNA (0.04 µM (micro M)), 1 × RNaseR Buffer, RNase R (0.1 units) or milliQ (control). An absorption spectrum at 260 nm was measured at 37°C for 1 hour using UV/VIS Spectrophotometer V-560 (JASCO). This was repeated three times to obtain N = 3. In this procedure, decomposition of the target nucleic acid can be detected as the UV absorption value increases.

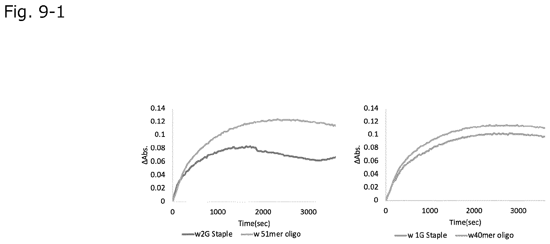

The results are shown in Figure 9-1 (the left chart of Figure 9-1 shows results for the second generation-type Staple nucleic acid, and the right chart of Figure 9-1 shows results for the first generation-type Staple nucleic acid). It could be confirmed that, by introduction of the first generation-type Staple nucleic acid or the second generation-type Staple nucleic acid, formation of a guanine quadruplex structure in "NF1 3'UTR" on the 3' end side of the target nucleic acid was induced, and even in the presence of RNaseR, the resistance of the target nucleic acid to 3'→5' exonuclease activity was improved, and the stability of the target nucleic acid was improved (Figure 9-1). Further, in the case of the combination of the target nucleic acid and the Staple nucleic acid, the second generation-type Staple nucleic acid improves the stability of the target nucleic acid more than the first generation-type Staple nucleic acid does.

### (7-2) Protein expression level upregulating action

In this Example, the ability of the Staple nucleic acid of the present invention to form a guanine quadruplex structure in a target nucleic acid even in cells, and whether, as a result, the stability of the target nucleic acid is improved (hence, the protein expression is upregulated) even in the cells were confirmed.

In this Example, the sequence of NF1 used in (7-1) above was used as a target nucleic acid, and the second generation-type Staple nucleic acid was used as a Staple nucleic acid.

MCF-7 #9 cells were seeded on a 10 cm dish at 1.0 × 10⁵ cells/ml, and cultured for 72 hours in 10 ml of E-MEM medium containing 10% FBS and 1% penicillin. When the confluency was confirmed to be 80%, the medium was changed to 10 ml of E-MEM medium containing 10% FBS, followed by culture for 3 hours.

The cells were divided into two groups, a group to which the Staple oligonucleotide was to be applied and a group to which the Staple oligonucleotide was not to be applied. In accordance with the protocol of FuGENE (registered trademark) HD Transfection Reagent (Promega E2311), 17 µg (microgram) of pIRES which expresses the sequence of the Staple oligonucleotide was added to the medium for the former group, and 17 µg (microgram) of a vector solution of blank pIRES free of inserts was added to the medium for the latter group. Thereafter, the cells were cultured in a 5% CO₂ atmosphere at 37°C for 48 hours.

After the cells were cultured for 48 hours, the medium was removed, and the cells were washed twice with 5 ml of D-PBS(-). 350 µl (microliter) of a solution obtained by mixing RIPA buffer (Nacalai Tesque) and a protease inhibitor cocktail (Promega) at 99 : 1 was applied to the dish, and the cells were collected using a cell scraper. The cell lysate was passed through a 25 G needle ten times, and centrifuged at 4°C for 10 minutes. The supernatant was transferred into a new tube, and mixed with a 6 × SDS sample buffer (Nacalai Tesque), and the mixture was heated at 95°C for 5 minutes.

The sample was separated by performing electrophoresis at 125 V and 126 mA for 110 minutes with Mini-PROTEAN TGX Gels (BIO-RAD), and blotted.

A solution obtained by diluting a primary antibody against NF1 (Neurofibromin 1 D7R7D Rabbit mAb #14623: Cell Signaling Technology) or a primary antibody against Vinculin (Vinculin Recombinant Rabbit Monoclonal Antibody 42H89L44: Invitrogen) as a control to 1/1,000 with Signal Enhancer HIKARI for Western Blotting and ELISA (Nacalai Tesque) was applied to a membrane blotted with the protein, which was shaken at room temperature for 90 minutes.

The membrane was washed with PBS, a solution obtained by diluting a secondary antibody against rabbit IgG (#7074 Anti-rabbit IgG, HRP-linked Antibody: Cell Signaling Technology) to 1/1,000 with Signal Enhancer HIKARI for Western Blotting and ELISA was then applied, which was shaken at room temperature for 60 minutes.

The membrane was washed with PBS, Chemi-Lumi One Super (Nacalai Tesque) was then applied to the membrane, which was left to stand at room temperature for 1 minute, followed by detection of a band with ImageQuant LAS 500 (GE healthcare).

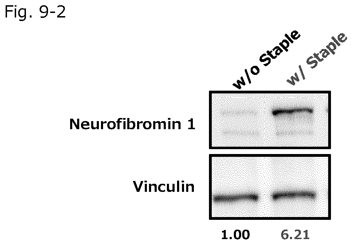

The results are shown in Figure 9-2. It was shown that, when the Staple nucleic acid was applied, the protein expression of NF1 which is a target nucleic acid considerably upregulated as compared to the case where the Staple nucleic acid was not applied.

### Example 8: Studies of Staple nucleic acid of invention in single target nucleic acid on ribosomal shunting action (2)

In this Example, it was confirmed that the first generation-type Staple nucleic acid or the second generation-type Staple nucleic acid was used to exert an action that causes ribosomal shunting over sub-sequences of a single target nucleic acid was generated.

In this Example, the sequence of a part of a known firefly luciferase (F Luc) gene was modified to design the nucleotide sequence of a modified F Luc gene as a template of mRNA (SEQ ID NO: 49) which is a target nucleic acid for the first generation-type Staple nucleic acid. The modified F Luc gene mRNA as a target nucleic acid was designed to enable production of functional F Luc protein only if ribosomal shunting occurs in the molecule of the target nucleic acid when the first generation-type Staple nucleic acid of the present invention hybridizes with the target nucleic acid to form a guanine quadruplex structure by the target nucleic acid and the Staple nucleic acid.

### [Chem. 29]

### <Modified F Luc (for first generation-type Staple nucleic acid)>

GUUUUCCCAGUCACGACGUUGUAAAACGACGGCCAGUGAAUUCGAGCUCGGUACCCGGG
UAAUACGACUCACUAUAGGGCCUUUUUGAUAAUCUCAUGACCAAAAAUGGCCGAUGCUA
AGAACAUUAAGAAGGGCCCTGCTCCCTTCTACCCTCTGGAGGATGGCACCGCTGGCGAGCA
GCTGCACAAGGCCATGAAGAGGTATGCCCTGGTGCCTGGCACCATTGCCTTCACCGATGCCC
ACATTGAGGTGGACATCACCTATGCCGAGTACTTCGAGATGTCTGTGCGCCTGGCCGAGGCC
ATGAAGAGGTACGGCCTGAACACCAACCACCGCATCGTGGTGTGCTCTGAGAACTCTCTGCA
GTTCTTCATGCCAGTGCTGGGCGCCCTGTTCATCGGAGTGGCCGTGGCCCCTGCTAACGACA
TTTACAACGAGCGGAGCTGCTGAACAGCATGGGCATTTCTCAGCCTACCGTGGTGTTCGTG
TCTAAGAAGGGCCTGCAGAAGATCCTGAACGTGCAGAAGAAGCTGCCTATCATCCAGAAGAT
CATCATCATGGACTCTAAGACCGACTACCAGGGCTTCCAGAGCATGTACACATTCGTGACATC
TCATCTGCCTCCTGGCTTCAACGAGTACGACTTCGTGCCAGAGTCTTTCGACAGGGACAAAAC
CATTGCCCTGATCATGAACAGCTCTGGGTCTACCGGCCTGCCTAAGGGCGTGGCCCTGCCTC
ATCGCACCGCCTGTGTGCGCTTCTCTCACGCCCGCGACCCTATTTTCGGCAACCAGATCATCC
CCGACACCGCTATTCTGAGCGTGGTGCCATTCCACCACGGCTTCGGCATGTTCACCACCCTG
GGCTACCTGATTTGCGGCUUAUUCCAUGGACUUCAUUUACUGGGUUUAACUUGGGUGGG
UGUGAAGAUAUUAGCAUACGCUACUGCAGUGGGUGGGUGUCGACCUAGAUUAAUGCAAU
UCGUACGAAGUUCAUAGCAUUUCCAGCACCCAAUUGAAGCUUUGGGUGGGUCAAGCUAU
UCAGCAGGUAUACAUAGUUGGAAAUCUCUGGAAGAUCCGCGCGUACCGAGUUCUAAUUC
ACUGGCCGUCGUUUUACUUUUGCCAGUAGGGCGGCGCCAUGGACUUCAUUUACUGGGU
UUAACUUCTTTCGGGTGGTGCTGATGTACCGCTTCGAGGAGGAGCTGTTCCTGCGCAGCCTG
CAAGACTACAAAATTCAGTCTGCCCTGCTGGTGCCAACCCTGTTCAGCTTCTTCGCTAAGAGC
ACCCTGATCGACAAGTACGACCTGTCTAACCTGCACGAGATTGCCTCTGGCGGCGCCCCACT
GTCTAAGGAGGTGGGCGAAGCCGTGGCCAAGCGCTTTCATCTGCCAGGCATCCGCCAGGGCT
ACGGCCTGACCGAGACAACCAGCGCCATTCTGATTACCCCAGAGGGCGACGACAAGCCTGGC
GCCGTGGGCAAGGTGGTGCCATTCTTCGAGGCCAAGGTGGTGGACCTGGACACCGGCAAGA
CCCTGGGAGTGAACCAGCGCGGCGAGCTGTGTGTGCGCGGCCCTATGATTATGTCCGGCTAC
GTGAATAACCCTGAGGCCACAAACGCCCTGATCGACAAGGACGGCTGGCTGCACTCTGGCGA
CATTGCCTACTGGGACGAGGACGAGCACTTCTTCATCGTGGACCGCCTGAAGTCTCTGATCA
AGTACAAGGGCTACCAGGTGGCCCCAGCCGAGCTGGAGTCTATCCTGCTGCAGCACCCTAAC
ATTTTCGACGCCGGAGTGGCCGGCCTGCCCGACGACGATGCCGGCGAGCTGCCTGCCGCCGT
CGTCGTGCTGGAACACGGCAAGACCATGACCGAGAAGGAGATCGTGGACTATGTGGCCAGCC
AGGTGACAACCGCCAAGAAGCTGCGCGGCGGAGTGGTGTTCGTGGACGAGGTGCCCAAGGG
CCTGACCGGCAAGCTGGACGCCCGCAAGATCCGCGAGATCCTGATCAAGGCTAAGAAAGGCG
GCAAGATCGCCGTGTAA (SEQ ID NO: 49)

Grayly shaded region: firefly luciferase sequence
Boxed region: the guanine repeat sequence used in formation of guanine quadruplex structure
Underlined region: the region where Staple nucleic acid is bound

In this Example, first generation-type Staple nucleic acids to be applied to the target nucleic acid were prepared as follows. Specifically, the first generation-type Staple nucleic acids prepared were two types of Staple nucleic acids containing a first nucleotide sequence and a second nucleotide sequence which hybridize at the underlined Staple nucleic acid binding regions of the nucleotide sequence of the target nucleic acid (Staple nucleic acid B3 (SEQ ID NO: 50) and Staple nucleic acid B4 (SEQ ID NO: 51)).

### [Chem. 30]

Staple nucleic acid B3
   AGUAGCGUAUGCUAAUAUCUUCACA CUGGCAAAAGUAAA (SEQ ID NO: 50)
Staple nucleic acid B4
   UAUGCUAAUAUCUUCACA CUGGCAAAAGUAAA (SEQ ID NO: 51)
Boxed region: the guanine repeat sequence used in formation of guanine quadruplex structure
Underlined region: the first nucleotide sequence and the second nucleotide sequence

In this Example, the nucleotide sequence of a modified F Luc gene as a template of mRNA (SEQ ID NO: 52) which is a target nucleic acid for the second generation-type Staple nucleic acid was also designed. The modified F Luc gene mRNA as a target nucleic acid was designed to enable production of functional F Luc protein only if ribosomal shunting occurs in the molecule of the target nucleic acid when the second generation-type Staple nucleic acid of the present invention hybridizes with the target nucleic acid to form a guanine quadruplex structure by the target nucleic acid and the Staple nucleic acid.

### [Chem. 31]

### <Modified F Luc (for second generation-type Staple nucleic acid)>

GUUUUCCCAGUCACGACGUUGUAAAACGACGGCCAGUGAAUUCGAGCUCGGUACCCGGG
UAAUACGACUCACUAUAGGGCCUUUUUGAUAAUCUCAUGACCAAAAAUGGCCGAUGCUA
AGAACAUUAAGAAGGGCCCTGCTCCCTTCTACCCTCTGGAGGATGGCACCGCTGGCGAGCA
GCTGCACAAGGCCATGAAGAGGTATGCCCTGGTGCCTGGCACCATTGCCTTCACCGATGCCC
ACATTGAGGTGGACATCACCTATGCCGAGTACTTCGAGATGTCTGTGCGCCTGGCCGAGGCC
ATGAAGAGGTACGGCCTGAACACCAACCACCGCATCGTGGTGTGCTCTGAGAACTCTCTGCA
GTTCTTCATGCCAGTGCTGGGCGCCCTGTTCATCGGAGTGGCCGTGGCCCCTGCTAACGACA
TTTACAACGAGCGGAGCTGCTGAACAGCATGGGCATTTCTCAGCCTACCGTGGTGTTCGTG
TCTAAGAAGGGCCTGCAGAAGATCCTGAACGTGCAGAAGAAGCTGCCTATCATCCAGAAGAT
CATCATCATGGACTCTAAGACCGACTACCAGGGCTTCCAGAGCATGTACACATTCGTGACATC
TCATCTGCCTCCTGGCTTCAACGAGTACGACTTCGTGCCAGAGTCTTTCGACAGGGACAAAAC
CATTGCCCTGATCATGAACAGCTCTGGGTCTACCGGCCTGCCTAAGGGCGTGGCCCTGCCTC
ATCGCACCGCCTGTGTGCGCTTCTCTCACGCCCGCGACCCTATTTTCGGCAACCAGATCATCC
CCGACACCGCTATTCTGAGCGTGGTGCCATTCCACCACGGCTTCGGCATGTTCACCACCCTG
GGCTACCTGATTTGCGGCUUAUUCCAUGGACUUCAUUUACUGGGUUUAACUUAAAUGGG
UGUGAAGAUAUUAGCAUACGCUACUGCAGUGGGUGGGUGUCGACCUAGAUUAAUGCAAU
UCGUACGAAGUUCAUAGCAUUUCCAGCACCCAAUUGAAGCUUUGGGUGGGUCAAGCUAU
UCAGCAGGUAUACAUAGUUGGAAAUCUCUGGAAGAUCCGCGCGUACCGAGUUCUAAUUC
ACUGGCCGUCGUUUUACUUUUGCCAGUAGGGCAACACCAUGGACUUCAUUUACUGGGUU
UAACUUCTTTCGGGTGGTGCTGATGTACCGCTTCGAGGAGGAGCTGTTCCTGCGCAGCCTGC
AAGACTACAAAATTCAGTCTGCCCTGCTGGTGCCAACCCTGTTCAGCTTCTTCGCTAAGAGCA
CCCTGATCGACAAGTACGACCTGTCTAACCTGCACGAGATTGCCTCTGGCGGCGCCCCACTG
TCTAAGGAGGTGGGCGAAGCCGTGGCCAAGCGCTTTCATCTGCCAGGCATCCGCCAGGGCTA

CGGCCTGACCGAGACAACCAGCGCCATTCTGATTACCCCAGAGGGCGACGACAAGCCTGGCG
CCGTGGGCAAGGTGGTGCCATTCTTCGAGGCCAAGGTGGTGGACCTGGACACCGGCAAGAC
CCTGGGAGTGAACCAGCGCGGCGAGCTGTGTGTGCGCGGCCCTATGATTATGTCCGGCTACG
TGAATAACCCTGAGGCCACAAACGCCCTGATCGACAAGGACGGCTGGCTGCACTCTGGCGAC
ATTGCCTACTGGGACGAGGACGAGCACTTCTTCATCGTGGACCGCCTGAAGTCTCTGATCAA
GTACAAGGGCTACCAGGTGGCCCCAGCCGAGCTGGAGTCTATCCTGCTGCAGCACCCTAACA
TTTTCGACGCCGGAGTGGCCGGCCTGCCCGACGACGATGCCGGCGAGCTGCCTGCCGCCGTC
GTCGTGCTGGAACACGGCAAGACCATGACCGAGAAGGAGATCGTGGACTATGTGGCCAGCCA
GGTGACAACCGCCAAGAAGCTGCGCGGCGGAGTGGTGTTCGTGGACGAGGTGCCCAAGGGC
CTGACCGGCAAGCTGGACGCCCGCAAGATCCGCGAGATCCTGATCAAGGCTAAGAAAGGCG
GCAAGATCGCCGTGTAA (SEQ ID NO: 52)

Grayly shaded region: firefly luciferase sequence
Boxed region: guanine the repeat sequence used in formation of guanine quadruplex structure
Underlined region: the region where Staple nucleic acid is bound

In this Example, second generation-type Staple nucleic acids to be applied to the target nucleic acid were prepared as follows. Specifically, the second generation-type Staple nucleic acids prepared were one type of Staple nucleic acids containing: a first nucleotide sequence and a second nucleotide sequence which hybridize at the underlined Staple nucleic acid binding regions of the nucleotide sequence of the target nucleic acid; and two guanine repeat sequences (Staple nucleic acid B5 (SEQ ID NO: 53)).

### [Chem. 32]

### Staple nucleic acid B5

AGUAGCGUAUGCUAAUAUCUUCA CGGGUGGG UACUGGCAAAAGUAAA (SEQ ID NO: 53)

Boxed region: the guanine repeat sequence used in formation of guanine quadruplex structure
Underlined region: the first nucleotide sequence and the second nucleotide sequence

The modified F Luc gene mRNA (SEQ ID NO: 49 or SEQ ID NO: 52) was used as a template (1 µg (microgram)), with which 20 µL (microliter) of a cell-free protein expression mixed solution (RTS 100 Wheat Germ CECF Kit, 5 Prime, Inc) with or without 0.14 µM (micro M) of each Staple nucleic acid was reacted at 24°C for 150 minutes. The obtained RNA was used as a mRNA template (1 µg (microgram)), with which 20 µL (microliter) of a cell-free protein expression mixed solution (RTS 100 Wheat Germ CECF Kit, 5 Prime, Inc) with or without 0.14 µM (micro M) of the Staple nucleic acid was reacted at 24°C for 150 minutes. Fluorescence measurement was performed using a fluorescence spectrophotometer (Jasco FP-8500) under the condition of 25°C with the excitation wavelength set to 480 nm. A sample with F Luc expressed based on the nucleotide sequence of the F Luc gene was used as a positive control, and a sample with modified F Luc expressed without applying the Staple nucleic acid was used as a negative control.

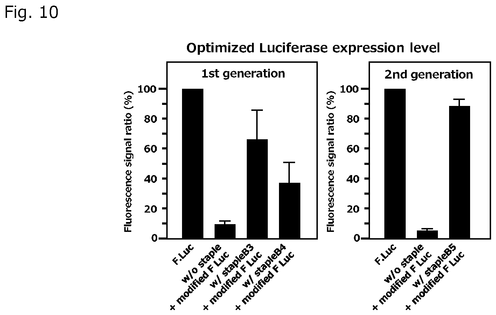

The results are shown in Figure 10. In this figure, the F Luc signal intensities of the modified F Luc expression sample (without Staple nucleic acid), the modified F Luc expression sample in the presence of the first generation-type Staple nucleic acid (with Staple nucleic acid B3 and Staple nucleic acid B4) (left chart) and the modified F Luc expression sample in the presence of the second generation-type Staple nucleic acid (with Staple nucleic acid B5) (right chart) are compared, where the F Luc signal intensity when F Luc is expressed based on the nucleotide sequence of the F Luc gene as a positive control is defined as 100. The results show that the F Luc signal intensity of the modified F Luc expression sample (without Staple nucleic acid) was 9.1% or 6.2% of that of the positive control, whereas the F Luc signal intensity when the first generation-type Staple nucleic acid was applied was 68.4% (with Staple nucleic acid B3) or 35.4% (with Staple nucleic acid B4), and the F Luc signal intensity when the second generation-type Staple nucleic acid was applied increased to 88.6% (Staple nucleic acid B5).

### Example 9: Studies of Staple nucleic acid of invention between two types of target nucleic acid on ribosomal shunting action

In this Example, it was confirmed that the first generation-type Staple nucleic acid and the second generation-type Staple nucleic acid were used to exert an action that causes ribosomal shunting over sub-sequences between two types target nucleic acids was generated.

The following scheme shows an outline of a method for confirming ribosomal shunting between two target nucleic acids, taking the first generation-type Staple nucleic acid as an example. Specifically, ribosomal shunting was examined by forming a guanine quadruplex structure by the Staple nucleic acid as in (1) between a fragment of gene A and a fragment of gene B, confirming whether ribosomes transferred from RNA of gene A to RNA of gene B at the location of the guanine quadruplex structure. The switching from RNA of gene A to RNA of gene B was confirmed by checking preparation of a functional nucleotide sequence due to linking between gene A and gene B when ribosomal shunting occurs at a relevant location. Note that (2) to (4) each functions as a control which cannot form a guanine quadruplex structure.

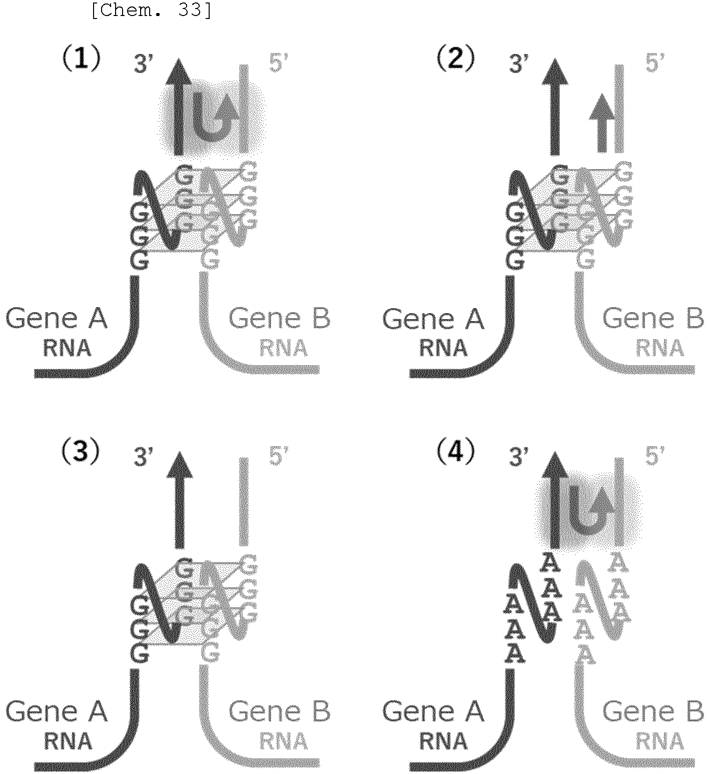

For examining whether the first generation-type Staple nucleic acid caused ribosomal shunting, the following sequences of gene A and gene B were designed as the target nucleic acids.

### [Chem. 34]

### <Gene A sequence (for first generation-type Staple nucleic acid)>

GUUUUCCCAGUCACGACGUUGUAAAACGACGGCCAGUGAAUUCGAGCUCGGUACCCGGG
UAAUACGACUCACUAUAGGGCCUUUUUGAUAAUCUCAUGACCAAAAAUGGCCGAUGCUA
AGAACAUUAAGAAGGGCCCTGCTCCCTTCTACCCTCTGGAGGATGGCACCGCTGGCGAGCA
GCTGCACAAGGCCATGAAGAGGTATGCCCTGGTGCCTGGCACCATTGCCTTCACCGATGCCC
ACATTGAGGTGGACATCACCTATGCCGAGTACTTCGAGATGTCTGTGCGCCTGGCCGAGGCC
ATGAAGAGGTACGGCCTGAACACCAACCACCGCATCGTGGTGUGCTCTGAGAACTCTCTGCA
GTTCTTCATGCCAGTGCTGGGCGCCCTGTTCATCGGAGTGGCCGTGGCCCCTGCTAACGACA
TTTACAACGAGCGGAGCTGCTGAACAGCATGGGCATTTCTCAGCCTACCGTGGTGTTCGTG
TCTAAGAAGGGCCTGCAGAAGATCCTGAACGTGCAGAAGAAGCTGCCTATCATCCAGAAGAT
CATCATCATGGACTCTAAGACCGACTACCAGGGCTTCCAGAGCATGTACACATTCGTGACATC
TCATCTGCCTCCTGGCTTCAACGAGTACGACTTCGTGCCAGAGTCTTTCGACAGGGACAAAAC
CATTGCCCTGATCATGAACAGCTCTGGGTCTACCGGCCTGCCTAAGGGCGTGGCCCTGCCTC
ATCGCACCGCCTGTGTGCGCTTCTCTCACGCCCGCGACCCTATTTTCGGCAACCAGATCATCC
CCGACACCGCTATTCTGAGCGTGGTGCCATTCCACCACGGCTTCGGCATGTTCACCACCCTG
GGCTACCTGATTTGCGGCUUAUUCCAUGGACUUCAUUUACUGGGUUUAACUUGGGUGGG
UGUGAAGAUAUUAGCAUACGCUACUGCAGUGGGUGGGUGUCGACCUAGAUUAAUGCAAU
UCGUACGAAGUUCAUAGCAUUUCCAGCACCCAAUUGAAGCUUUGGGUGGGU (SEQ ID NO:
54)

Boxed region: the guanine repeat sequence used in formation of guanine quadruplex structure
Gray region: sub-sequence of firefly luciferase gene
Underlined region: the region where first generation-type Staple nucleic acid is bound

### [Chem. 35]

### <Gene B sequence (for first generation-type Staple nucleic acid)>

guggcacauugugccacGCAUGCCAUGACUUGCAAGCUAUUCAGCAGGUAUACAUAGUUGGAAA
UCUCUGGAAGAUCCGCGCGUACCGAGUUCUAAUUCACUGGCCGUCGUUUUACUUUUGCC
AGUAGGGCGGCGCCAUGGACUUCAUUUACUGGGUUUAACUUCTTTCGGGTGGTGCTGAT
GTACCGCTTCGAGGAGGAGCTGTTCCTGCGCAGCCTGCAAGACTACAAAATTCAGTCTGCCC
TGCTGGTGCCAACCCTGTTCAGCTTCTTCGCTAAGAGCACCCTGATCGACAAGTACGACCTGT
CTAACCTGCACGAGATTGCCTCTGGCGGCGCCCCACTGTCTAAGGAGGTGGGCGAAGCCGTG
GCCAAGCGCTTTCATCTGCCAGGCATCCGCCAGGGCTACGGCCTGACCGAGACAACCAGCGC
CATTCTGATTACCCCAGAGGGCGACGACAAGCCTGGCGCCGTGGGCAAGGTGGTGCCATTCT
TCGAGGCCAAGGTGGTGGACCTGGACACCGGCAAGACCCTGGGAGTGAACCAGCGCGGCGA
GCTGTGTGTGCGCGGCCCTATGATTATGTCCGGCTACGTGAATAACCCTGAGGCCACAAACG
CCCTGATCGACAAGGACGGCTGGCTGCACTCTGGCGACATTGCCTACTGGGACGAGGACGAG
CACTTCTTCATCGTGGACCGCCTGAAGTCTCTGATCAAGTACAAGGGCTACCAGGTGGCCCC
AGCCGAGCTGGAGTCTATCCTGCTGCAGCACCCTAACATTTTCGACGCCGGAGTGGCCGGCC
TGCCCGACGACGATGCCGGCGAGCTGCCTGCCGCCGTCGTCGTGCTGGAACACGGCAAGAC
CATGACCGAGAAGGAGATCGTGGACTATGTGGCCAGCCAGGTGACAACCGCCAAGAAGCTGC
GCGGCGGAGTGGTGTTCGTGGACGAGGTGCCCAAGGGCCTGACCGGCAAGCTGGACGCCCG
CAAGATCCGCGAGATCCTGATCAAGGCTAAGAAAGGCGGCAAGATCGCCGTGTAA (SEQ ID
NO: 55)

Boxed region: the guanine repeat sequence used in formation of guanine quadruplex structure
Gray region: sub-sequence of firefly luciferase gene
Underlined region: the region where first generation-type Staple nucleic acid is bound

Further in this Example, for these target nucleic acids, a first generation-type Staple nucleic acid for use in the method (1) and a control nucleic acid for use in the method (2) were created as follows.

### [Chem. 36]

### Staple nucleic acid C1 (first generation-type Staple nucleic acid)

AGUAGCGUAUGCUAAUAUCUUCAC ACUGGCAAAAGUAAA (SEQ ID NO: 56)
Boxed region: the guanine repeat sequence used in formation of guanine quadruplex structure
Underlined region: the first nucleotide sequence and the second nucleotide sequence

### Control nucleic acid C2

ACUGGCAAAAGUAAA (SEQ ID NO: 57)
Boxed region: the guanine repeat sequence used in formation of guanine quadruplex structure
Underlined region: the first nucleotide sequence and the second nucleotide sequence

For examining whether the second generation-type Staple nucleic acid caused ribosomal shunting, the following sequences of gene A and gene B were designed as the target nucleic acids.

### [Chem. 37]

### <Gene A sequence (for second generation-type Staple nucleic acid)>

GUUUUCCCAGUCACGACGUUGUAAAACGACGGCCAGUGAAUUCGAGCUCGGUACCCGGG
UAAUACGACUCACUAUAGGGCCUUUUUGAUAAUCUCAUGACCAAAAAUGGCCGAUGCUA
AGAACAUUAAGAAGGGCCCTGCTCCCTTCTACCCTCTGGAGGATGGCACCGCTGGCGAGCA
GCTGCACAAGGCCATGAAGAGGTATGCCCTGGTGCCTGGCACCATTGCCTTCACCGATGCCC
ACATTGAGGTGGACATCACCTATGCCGAGTACTTCGAGATGTCTGTGCGCCTGGCCGAGGCC
ATGAAGAGGTACGGCCTGAACACCAACCACCGCATCGTGGTGTGCTCTGAGAACTCTCTGCA
GTTCTTCATGCCAGTGCTGGGCGCCCTGTTCATCGGAGTGGCCGTGGCCCCTGCTAACGACA
TTTACAACGAGCGCGAGCTGCTGAACAGCATGGGCATTTCTCAGCCTACCGTGGTGTTCGTG
TCTAAGAAGGGCCTGCAGAAGATCCTGAACGTGCAGAAGAAGCTGCCTATCATCCAGAAGAT
CATCATCATGGACTCTAAGACCGACTACCAGGGCTTCCAGAGCATGTACACATTCGTGACATC
TCATCTGCCTCCTGGCTTCAACGAGTACGACTTCGTGCCAGAGTCTTTCGACAGGGACAAAAC
CATTGCCCTGATCATGAACAGCTCTGGGTCTACCGGCCTGCCTAAGGGCGTGGCCCTGCCTC
ATCGCACCGCCTGTGTGCGCTTCTCTCACGCCCGCGACCCTATTTTCGGCAACCAGATCATCC
CCGACACCGCTATTCTGAGCGTGGTGCCATTCCACCACGGCTTCGGCATGTTCACCACCCTG
GGCTACCTGATTTGCGGCUUAUUCCAUGGACUUCAUUUACUGGGUUUAACUUAAAUGGG
UGUGAAGAUAUUAGCAUACGCUACUGCAGUGGGUGGGUGUCGACCUAGAUUAAUGCAAU
UCGUACGAAGUUCAUAGCAUUUCCAGCACCCAAUUGAAGCUUUGGGUGGGU (SEQ ID NO:
58)

Boxed region: the guanine repeat sequence used in formation of guanine quadruplex structure
Gray region: sub-sequence of firefly luciferase gene
Underlined region: the region where second generation-type Staple nucleic acid is bound

### [Chem. 38]

### <Gene B sequence (for second generation-type Staple nucleic acid)>

guggcacauugugccacGCAUGCCAUGACUUGCAAGCUAUUCAGCAGGUAUACAUAGUUGGAAA
UCUCUGGAAGAUCCGCGCGUACCGAGUUCUAAUUCACUGGCCGUCGUUUUACUUUUGCC
AGUAGGGCAACACCAUGGACUUCAUUUACUGGGUUUAACUUCTTTCGGGTGGTGCTGATG
TACCGCTTCGAGGAGGAGCTGTTCCTGCGCAGCCTGCAAGACTACAAAATTCAGTCTGCCCT
GCTGGTGCCAACCCTGTTCAGCTTCTTCGCTAAGAGCACCCTGATCGACAAGTACGACCTGTC
TAACCTGCACGAGATTGCCTCTGGCGGCGCCCCACTGTCTAAGGAGGTGGGCGAAGCCGTGG
CCAAGCGCTTTCATCTGCCAGGCATCCGCCAGGGCTACGGCCTGACCGAGACAACCAGCGCC

ATTCTGATTACCCCAGAGGGCGACGACAAGCCTGGCGCCGTGGGCAAGGTGGTGCCATTCTT
CGAGGCCAAGGTGGTGGACCTGGACACCGGCAAGACCCTGGGAGTGAACCAGCGCGGCGAG
CTGTGTGTGCGCGGCCCTATGATTATGTCCGGCTACGTGAATAACCCTGAGGCCACAAACGC
CCTGATCGACAAGGACGGCTGGCTGCACTCTGGCGACATTGCCTACTGGGACGAGGACGAGC
ACTTCTTCATCGTGGACCGCCTGAAGTCTCTGATCAAGTACAAGGGCTACCAGGTGGCCCCA
GCCGAGCTGGAGTCTATCCTGCTGCAGCACCCTAACATTTTCGACGCCGGAGTGGCCGGCCT
GCCCGACGACGATGCCGGCGAGCTGCCTGCCGCCGTCGTCGTGCTGGAACACGGCAAGACC
ATGACCGAGAAGGAGATCGTGGACTATGTGGCCAGCCAGGTGACAACCGCCAAGAAGCTGC
GCGGCGGAGTGGTGTTCGTGGACGAGGTGCCCAAGGGCCTGACCGGCAAGCTGGACGCCCG
CAAGATCCGCGAGATCCTGATCAAGGCTAAGAAAGGCGGCAAGATCGCCGTGTAA (SEQ ID
NO: 59)

Boxed region: the guanine repeat sequence used in formation of guanine quadruplex structure
Gray region: sub-sequence of firefly luciferase gene
Underlined region: the region where first generation-type Staple nucleic acid is bound

Further in this Example, for these target nucleic acids, a second generation-type Staple nucleic acid (Staple nucleic acid C3) for use in the methods (1) and (4), and a control nucleic acid (control nucleic acid C4) for use in the method (2) were created as follows.

### [Chem. 39]

### Staple nucleic acid C3 (second generation-type Staple nucleic acid)

AGUAGCGUAUGCUAAUAUCUUCACGGGUGGGUACUGGCAAAAGUAAA (SEQ ID NO: 60)

Boxed region: the guanine repeat sequence used in formation of guanine quadruplex structure
Underlined region: the first nucleotide sequence and the second nucleotide sequence

### Control nucleic acid C4

GGGUACUGGCAAAAGUAAA (SEQ ID NO: 61)

Boxed region: the guanine repeat sequence used in formation of guanine quadruplex structure
Underlined region: the first nucleotide sequence and the second nucleotide sequence

The gene A and the gene B were mixed at a ratio of 1 : 1 on a concentration basis, 1.5 equivalents of the Staple nucleic acid was added with respect to the mixture, and the temperature was lowered at 1°C per minute from 90°C to perform annealing. Here, as the first generation-type Staple nucleic acid, the Staple nucleic acid C1 was used for the condition (1), the Staple nucleic acid C2 was used for the condition (2), the Staple nucleic acid was not added for the condition (3), and the Staple nucleic acid C1 was used for the condition (4).

A total of 1 µg (microgram) of the complex mRNA was used as mRNA (1 µg (microgram)) in 12.5 µl (microliter) of a cell-free protein synthesis reaction solution (RTS 100 Wheat Germ Kit, biotechrabbit). This reaction solution was incubated at 24°C for 1 hour, and then evaluated for luciferase activity using a Luciferase Assay kit (Promega) and a POWERSCAN-H1 microplate reader (BioTek).

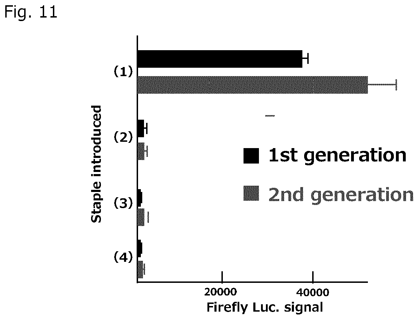

The results are shown in Figure 11. This figure demonstrates that in the case of the first generation-type Staple nucleic acid as well as the second generation-type Staple nucleic acid, fluorescence of luciferase was hardly detected in (2) to (4), whereas fluorescence of luciferase was highly detected in (1). The results showed that, when a guanine quadruplex structure was formed between two molecules using the Staple nucleic acid of the present invention, ribosomal shunting between the two molecules occurred at the location of the guanine quadruplex structure.

### Industrial Applicability

The present invention can develop and provide a novel Staple nucleic acid (second generation-type Staple nucleic acid) which can supply guanine repeat sequences via an oligonucleotide, so that a guanine quadruplex structure can be formed on a target nucleic acid by a total of four guanine repeat sequences consisting of the guanine repeat sequences on the target nucleic acid and the guanine repeat sequences on the oligonucleotide. The present invention can also provide novel use applications of Staple nucleic acids for the first generation-type Staple nucleic acids and that of the second generation-type Staple nucleic acids.

## Claims

1. A G supply-type oligonucleotide comprising: a first nucleotide sequence and a second nucleotide sequence, which target a nucleotide sequence portion containing one to three guanine repeat sequences on a target nucleic acid, and each hybridize with a nucleotide sequence on the 5' side or 3' side of the guanine repeat sequences; and three to one guanine repeat sequences depending on the number of guanine repeat sequences on the target nucleic acid,
wherein the G supply-type oligonucleotide changes the conformation of the target nucleic acid through hybridization of the first nucleotide sequence and the second nucleotide sequence with the target nucleic acid, decreases a spatial distance between a total of four guanine repeat sequences consisting of the guanine repeat sequences on the target nucleic acid and the guanine repeat sequences on the G supply-type oligonucleotide, to form a guanine quadruplex structure composed of the four guanine repeat sequences.

2. The G supply-type oligonucleotide according to claim 1, wherein the first nucleotide sequence and the second nucleotide sequence of the G supply-type oligonucleotide hybridize with
- a nucleotide sequence portion on the 5' side or 3' side of a guanine repeat sequence on the 3' terminal side of the guanine repeat sequences on the target nucleic acid, and
- a nucleotide sequence portion on the 5' side or 3' side of a guanine repeat sequence on the 5' terminal side of the guanine repeat sequences on the target nucleic acid.

3. The G supply-type oligonucleotide according to claim 1 or 2, which is composed of DNA, RNA, a modified nucleic acid, or a combination thereof.

4. A pharmaceutical composition comprising a G supply-type oligonucleotide comprising: a first nucleotide sequence and a second nucleotide sequence, which target a nucleotide sequence portion containing one to three guanine repeat sequences on a target nucleic acid, and each hybridize with a nucleotide sequence on the 5' side or 3' side of the guanine repeat sequences; and three to one guanine repeat sequences depending on the number of guanine repeat sequences on the target nucleic acid,
wherein the G supply-type oligonucleotide changes the conformation of the target nucleic acid through hybridization of the first nucleotide sequence and the second nucleotide sequence with the target nucleic acid, decreases a spatial distance between a total of four guanine repeat sequences consisting of the guanine repeat sequences on the target nucleic acid and the guanine repeat sequences on the G supply-type oligonucleotide, to form a guanine quadruplex structure composed of the four guanine repeat sequences.

5. The pharmaceutical composition according to claim 4, wherein the first nucleotide sequence and the second nucleotide sequence of the G supply-type oligonucleotide hybridize with
- a nucleotide sequence portion on the 5' side or 3' side of a guanine repeat sequence on the 3' terminal side of the guanine repeat sequences on the target nucleic acid, and
- a nucleotide sequence portion on the 5' side or 3' side of a guanine repeat sequence on the 5' terminal side of the guanine repeat sequences on the target nucleic acid.

6. The pharmaceutical composition according to claim 4 or 5, wherein the G supply-type oligonucleotide is composed of DNA, RNA, a modified nucleic acid, or a combination thereof.

7. A method for producing a G supply-type oligonucleotide comprising: a first nucleotide sequence and a second nucleotide sequence, which target a nucleotide sequence portion containing one to three guanine repeat sequences on a target nucleic acid, and each hybridize with a nucleotide sequence on the 5' side or 3' side of the guanine repeat sequences; and three to one guanine repeat sequences depending on the number of guanine repeat sequences on the target nucleic acid,
the method comprising designing and synthesizing the G supply-type oligonucleotide such that the first nucleotide sequence and the second nucleotide sequence of the G supply-type oligonucleotide hybridize with the target nucleic acid, and the G supply-type oligonucleotide is folded so as to decrease a spatial distance between a total of four guanine repeat sequences consisting of the guanine repeat sequences on the target nucleic acid and the guanine repeat sequences on the G supply-type oligonucleotide.

8. The method for producing a G supply-type oligonucleotide according to claim 7, wherein the first nucleotide sequence and the second nucleotide sequence of the G supply-type oligonucleotide hybridize with
- a nucleotide sequence portion on the 5' side or 3' side of a guanine repeat sequence on the 3' terminal side of the guanine repeat sequences on the target nucleic acid, and
- a nucleotide sequence portion on the 5' side or 3' side of a guanine repeat sequence on the 5' terminal side of the guanine repeat sequences on the target nucleic acid.

9. The method for producing a G supply-type oligonucleotide according to claim 7 or 8, wherein the G supply-type oligonucleotide is composed of DNA, RNA, a modified nucleic acid, or a combination thereof.

10. A method for downregulating protein expression from a target nucleic acid, using a G supply-type oligonucleotide comprising: a first nucleotide sequence and a second nucleotide sequence, which target a nucleotide sequence portion containing one to three guanine repeat sequences on a target nucleic acid, and each hybridize with a nucleotide sequence on the 5' side or 3' side of the guanine repeat sequences; and three to one guanine repeat sequences depending on the number of guanine repeat sequences on the target nucleic acid,
to change the conformation of the target nucleic acid through hybridization of the first nucleotide sequence and the second nucleotide sequence with the target nucleic acid, to decrease a spatial distance between a total of four guanine repeat sequences consisting of the guanine repeat sequences on the target nucleic acid and the guanine repeat sequences on the G supply-type oligonucleotide, to form a guanine quadruplex structure composed of the four guanine repeat sequences, by the G supply-type oligonucleotide.

11. The method for downregulating protein expression according to claim 10, wherein the first nucleotide sequence and the second nucleotide sequence of the G supply-type oligonucleotide hybridize with
- a nucleotide sequence portion on the 5' side or 3' side of a guanine repeat sequence on the 3' terminal side of the guanine repeat sequences on the target nucleic acid, and
- a nucleotide sequence portion on the 5' side or 3' side of a guanine repeat sequence on the 5' terminal side of the guanine repeat sequences on the target nucleic acid.

12. The method for downregulating protein expression according to claim 10 or 11, wherein the G supply-type oligonucleotide is composed of DNA, RNA, a modified nucleic acid, or a combination thereof.

13. The method for downregulating protein expression according to claim 10 or 11, wherein the downregulation of protein expression occurs through suppression of reverse transcription reaction or suppression of protein translation reaction.

14. A kit for downregulating protein expression comprising a G supply-type oligonucleotide comprising: a first nucleotide sequence and a second nucleotide sequence, which target a nucleotide sequence portion containing one to three guanine repeat sequences on a target nucleic acid, and each hybridize with a nucleotide sequence on the 5' side or 3' side of the guanine repeat sequences; and three to one guanine repeat sequences depending on the number of guanine repeat sequences on the target nucleic acid,
wherein the G supply-type oligonucleotide downregulates protein expression from the target nucleic acid by changing the conformation of the target nucleic acid through hybridization of the first nucleotide sequence and the second nucleotide sequence with the target nucleic acid, decreasing a spatial distance between a total of four guanine repeat sequences consisting of the guanine repeat sequences on the target nucleic acid and the guanine repeat sequences on the G supply-type oligonucleotide, to form a guanine quadruplex structure composed of the four guanine repeat sequences.

15. The kit for downregulating protein expression according to claim 14, wherein the first nucleotide sequence and the second nucleotide sequence of the G supply-type oligonucleotide hybridize with
- a nucleotide sequence portion on the 5' side or 3' side of a guanine repeat sequence on the 3' terminal side of the guanine repeat sequences on the target nucleic acid, and
- a nucleotide sequence portion on the 5' side or 3' side of a guanine repeat sequence on the 5' terminal side of the guanine repeat sequences on the target nucleic acid.

16. The kit for downregulating protein expression according to claim 14 or 15, wherein the downregulation of protein expression occurs through suppression of reverse transcription reaction or suppression of protein translation reaction.

17. A method for producing a modified protein from a target nucleic acid using an oligonucleotide comprising: a first nucleotide sequence and a second nucleotide sequence, which target a nucleotide sequence portion containing one to four guanine repeat sequences on a target nucleic acid, and each hybridize with a nucleotide sequence on the 5' side or 3' side of the guanine repeat sequences; and three to zero guanine repeat sequences depending on the number of guanine repeat sequences on the target nucleic acid,
wherein the oligonucleotide forms a loop portion on a part of the target nucleic acid, by changing the conformation of the target nucleic acid through hybridization of the first nucleotide sequence and the second nucleotide sequence with the target nucleic acid, decreasing a spatial distance between a total of four guanine repeat sequences consisting of guanine repeat sequences on the target nucleic acid and guanine repeat sequences on the oligonucleotide, to form a guanine quadruplex structure composed of the four guanine repeat sequences, and ribosomes shunt over the loop portion.

18. The method for producing a modified protein according to claim 17, wherein the first nucleotide sequence and the second nucleotide sequence of the oligonucleotide hybridize with
- a nucleotide sequence portion on the 5' side or 3' side of a guanine repeat sequence on the 3' terminal side of the guanine repeat sequences on the target nucleic acid, and
- a nucleotide sequence portion on the 5' side or 3' side of a guanine repeat sequence on the 5' terminal side of the guanine repeat sequences on the target nucleic acid.

19. The method for producing a modified protein according to claim 17 or 18, wherein the oligonucleotide is composed of DNA, RNA, a modified nucleic acid, or a combination thereof.

20. A method for producing a fused protein of a translated product fused with a sub-sequence of a first target nucleic acid and a sub-sequence of a second target nucleic acid, using an oligonucleotide comprising:
a first nucleotide sequence which targets a nucleotide sequence portion containing one to two guanine repeat sequences on the first target nucleic acid, and hybridizes with a nucleotide sequence on the 5' side or 3' side of the guanine repeat sequences,
a second nucleotide sequence which targets a nucleotide sequence portion containing one to two guanine repeat sequences on the second target nucleic acid, and hybridizes with a nucleotide sequence on the 5' side or 3' side of the guanine repeat sequences, and
two to zero guanine repeat sequences depending on the number of the guanine repeat sequences on the first target nucleic acid and the number of the guanine repeat sequences on the second target nucleic acid,
wherein the oligonucleotide hybridizes with the first target nucleic acid through its first nucleotide sequence and with the second target nucleic acid through its second nucleotide sequence, changes the conformation of the target nucleic acids, decreases a spatial distance between a total of four guanine repeat sequences consisting of one to two guanine repeat sequences on the first target nucleic acid, one to two guanine repeat sequences on the second target nucleic acid and two to zero guanine repeat sequences on the oligonucleotide, to form a guanine quadruplex structure composed of the four guanine repeat sequences, and ribosomes shunt between the first target nucleic acid and the second target nucleic acid.

21. The method for producing a fused protein according to claim 20,
wherein the first nucleotide sequence and the second nucleotide sequence of the oligonucleotide hybridize with
- a nucleotide sequence portion on the 5' side or 3' side of a guanine repeat sequence on the 3' terminal side of the guanine repeat sequences on the target nucleic acid, and
- a nucleotide sequence portion on the 5' side or 3' side of a guanine repeat sequence on the 5' terminal side of the guanine repeat sequences on the target nucleic acid.

22. The method for producing a fused protein according to claim 20 or 21, wherein the oligonucleotide is composed of DNA, RNA, a modified nucleic acid, or a combination thereof.

23. A method for stabilizing a target nucleic acid, using an oligonucleotide comprising: a first nucleotide sequence and a second nucleotide sequence which target a nucleotide sequence portion containing one to four guanine repeat sequences on a target nucleic acid, and each hybridize with a nucleotide sequence on the 5' side or 3' side of the guanine repeat sequences; and three to zero guanine repeat sequences depending on the number of guanine repeat sequences on the target nucleic acid,
wherein the oligonucleotide inhibits binding of a nucleolytic enzyme to the target nucleic acid or inhibits functions of the nucleolytic enzyme by changing the conformation of the target nucleic acid through hybridization of the first nucleotide sequence and the second nucleotide sequence with the target nucleic acid, decreasing a spatial distance between a total of four guanine repeat sequences consisting of guanine repeat sequences on the target nucleic acid and guanine repeat sequences on the oligonucleotide, to form a guanine quadruplex structure composed of the four guanine repeat sequences.

24. The method for stabilizing a target nucleic acid according to claim 23, wherein the first nucleotide sequence and the second nucleotide sequence of the oligonucleotide hybridize with
- a nucleotide sequence portion on the 5' side or 3' side of a guanine repeat sequence on the 3' terminal side of the guanine repeat sequences on the target nucleic acid, and
- a nucleotide sequence portion on the 5' side or 3' side of a guanine repeat sequence on the 5' terminal side of the guanine repeat sequences on the target nucleic acid.

25. The method for stabilizing a target nucleic acid according to claim 23 or 24, wherein the oligonucleotide is composed of DNA, RNA, a modified nucleic acid, or a combination thereof.
